# EUROPÄISCHE PATENTSCHRIFT

(11) **EP 3 500 568 B1**
(45) Veröffentlichungstag und Bekanntmachung des Hinweises auf die Patenterteilung: **23.09.2020**
(21) Anmeldenummer: 17746524.2
(22) Anmeldetag: 09.08.2017
(51) Int. Cl.: C07D 471/04, C07D 487/04, C07D 491/056, A01N 43/50, A01N 43/40

(54) **VERFAHREN ZUR HERSTELLUNG VON 2-(3,6-DIHALOPYRIDIN-2-YL)-3H-IMIDAZOL[4,5-C]PYRIDINDERIVATEN UND VERWANDTEN VERBINDUNGEN DURCH UMSETZUNG DES 3H-IMIDAZOL[4,5-C]PYRIDINDERIVATS MIT EINER METALLORGANISCHEN ZINK-AMIN BASE**
PROCESS FOR THE PREPARATION OF 2-(3,6-DIHALOPYRIDIN-2-YL)-3H-IMIDAZOL[4,5-C]PYRIDINE DERIVATIVES AND RELATED COMPOUNDS BY REACTING THE 3H-IMIDAZOL[4,5-C]PYRIDINE DERIVATIVE WITH A METAL-ORGANIC ZINC-AMINE BASE
PROCÉDÉ POUR LA PRÉPARATION DE DÉRIVÉS DE LA 2-(3,6-DIHALOPYRIDIN-2-YL)-3H-IMIDAZOL[4,5-C]PYRIDINE ET COMPOSÉS SIMILAIRES PAR REACTION D'UN DÉRIVÉ DE LA 3H-IMIDAZOL[4,5-C]PYRIDINE AVEC UNE BASE METAL-ORGANIC D'UNE AMINE DE ZINC

(30) Priorität: 16.08.2016 EP 16184368
(43) Veröffentlichungstag der Anmeldung: 26.06.2019
(73) Patentinhaber: Bayer CropScience Aktiengesellschaft, 40789 Monheim am Rhein (DE)
(72) Erfinder: MOSRIN, Marc, 50935 Köln (DE); FISCHER, Rüdiger, 50259 Pulheim (DE); HAGER, Dominik, 40789 Monheim (DE); HOFFMEISTER, Laura, 40593 Düsseldorf (DE); KAUSCH-BUSIES, Nina, 51467 Bergisch Gladbach (DE); WILCKE, David, 40235 Düsseldorf (DE); WILLOT, Matthieu, 40215 Düsseldorf (DE)
(74) Vertreter: BIP Patents
(86) Internationale Anmeldenummer: PCT/EP2017/070184
(87) Internationale Veröffentlichungsnummer: WO 2018/033448

(56) Entgegenhaltungen:
- WO-A1-2007/075567
- WO-A1-2016/124557
- WO-A2-2011/027249
- ROBERT J. WILSON ET AL: "Copper- and Palladium-Catalyzed Amidation Reactions for the Synthesis of Substituted Imidazo[4,5- c ]pyridines", THE JOURNAL OF ORGANIC CHEMISTRY, Bd. 79, Nr. 5, 7. März 2014 (2014-03-07), Seiten 2203-2212, XP055113503, ISSN: 0022-3263, DOI: 10.1021/jo500064j
- G. B. BARLIN: "Ionisation constants of heterocyclic substances. Part VIII. 1,3,5-Triazaindenes", JOURNAL OF THE CHEMICAL SOCIETY B: PHYSICAL ORGANIC, Bd. 0, 1966, Seiten 285-291, XP002762031, DOI: 10.1039/J29660000285
- HERZ, HANS-GEORG; QUEIROZ, MARIA JOAO R. P.; MAAS, GERHARD: "2-Arylvinylation of 1-methylindole by palladium-catalyzed cross-coupling reactions", SYNTHESIS, Bd. 6, 31. Dezember 1999 (1999-12-31), Seiten 1013-1016, XP002762032, DOI: 10.1055/s-1999-3506
- HAMMANN, JEFFREY M.; HAAS, DIANA; KNOCHEL, PAUL: "Cobalt-catalyzed Negishi cross-coupling reactions of (hetero)arylzinc reagents with primary and secondary alkyl bromides and iodides", ANGEWANDTE CHEMIE, INTERNATIONAL EDITION, Bd. 54, Nr. 15, 18. Februar 2015 (2015-02-18), Seiten 4478-4481, XP002762033, ISSN: 1433-7851, DOI: 10.1002/anie.201411960
- YOKOYAMA, MASATAKA; TOYOSHIMA, HIROFUMI; SHIMIZU, MIYUKI; TOGO, HIDEO: "Stereoselective coupling of riboses with metallic salts of aromatic heterocycles", JOURNAL OF THE CHEMICAL SOCIETY, PERKIN TRANSACTIONS 1: ORGANIC AND BIO-ORGANIC CHEMISTRY, Bd. 1, 1. Januar 1997 (1997-01-01), Seiten 29-33, XP002762034, ISSN: 0300-922X

## Beschreibung

Die vorliegende Erfindung betrifft ein Verfahren zur Herstellung von halogenierten Pyridinderivaten der Formel (II) 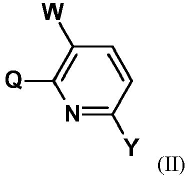 ausgehend von Verbindungen Q-H über Zwischenstufen der Formel (IIIa) oder (IIIb) 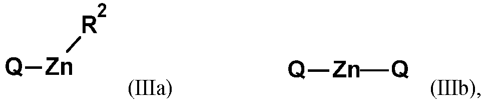 in denen die in den Formeln (II), (IIIa) und (IIIb) angegebenen Strukturelemente die nachstehend angegebenen Bedeutungen haben. Ferner betrifft die Erfindung derartige halogenierte Pyridinderivate und Zwischenstufen.

Halogenierte Pyridinderivate der Formel (II) haben große technische Bedeutung für die pharmazeutische und die agrochemische Industrie und sind eine wichtige Zwischenstufe unter anderem bei der Herstellung von Verbindungen, die beispielsweise als Pestizide wirksam sind.

In der Literatur ist bekannt, dass Verbindungen der Formel (II) beispielsweise in einem ersten Schritt durch Kondensation von Pyridin-2-carbonsäurederivaten mit ortho-substituierten Bis(amin)-, Aminalkohol- oder Aminthiol-(hetero)arylderivaten in Gegenwart eines Kondensationsmittels (vgl. US2003/69257 oder WO2006/65703) und anschließend in einem zweiten Schritt durch weitere Kondensation wie in WO2012/86848 beschrieben hergestellt werden können. Mittels zweifacher Kondensation werden in WO2011/027249 neue Benzimidazol-Derivate dargestellt, in WO2016/124557 bicyclische Heterocyclen-Derivate. Ebenfalls nicht katalytisch können auch 1,3,5-Triazaindene synthetisiert werden (G. B. Barlin et al., J. Chem. Soc. B, 1966, 285-291) und geschützte Ribose mit Magnesium-, Cadmium- und Zinksalzen aromatischer Heterocyclen gekuppelt werden (H. Togo et al., J. Chem. Soc. Perkin Trans. 1997, 1, 29-33).

In WO2007/075561 werden Herstellverfahren neuer Triazolpyridine mittels Übergangmetallkatalyse (Suzuki-, Stille- oder Negishi-Kupplung) offenbart. 2-Arylvinylierungen von 1-Methylindol sind mithilfe der Palladium-katalysierten Kreuzkupplung möglich (G. Maas et al., Synthesis 1999, 6, 1013-1016). (Hetero)arylzink-Reagenzien können mit Alkylbromiden und -iodiden in Cobalt-katalysierten Negishi-Kupplungen zur Reaktion gebracht werden (P. Knochel et al., Angew. Chem. Int. Ed. 2015, 54, 4478-4481). Mithilfe von Kupfer- und Palladium-katalysierten Amidierungen können Imidazo[4,5-c]pyridine dargestellt werden (R. J. Wilson et al., J. Org. Chem. 2014, 79, 2203-2212).

Die bisher im Stand der Technik beschriebenen chemischen Syntheseverfahren von derartigen halogenierten Pyridinderivaten bedienen sich aber sehr häufig Methoden, die aus industrieller Sicht wirtschaftlich nicht zu realisieren sind und/oder andere Nachteile aufweisen.

Nachteilig sind die geringen chemischen Ausbeuten, die Durchführung bei sehr hohen Temperaturen (ca. 150 bis 250°C) sowie die mögliche schwierige Regio- und Chemo-Selektivität der Kondensation, insbesondere bei Imidazopyridin- und Imidazopyridazinderivaten. Die Herstellung ist daher sehr teuer und nicht für großtechnische kommerzielle Verfahren geeignet. Außerdem sind entsprechende Verbindungen kaum kommerziell erhältlich. Dies gilt insbesondere für 3,6-Dihalogenpyridin-2-carbonsäurederivate.

Im Hinblick auf die vorstehend geschilderten Nachteile besteht dringend Bedarf für ein vereinfachtes, technisch und ökonomisch durchführbares Verfahren zur Herstellung von halogenierten Pyridinderivaten, insbesondere von halogenierten Pyridinderivaten der Formel (II). Die mit diesem angestrebten Verfahren erhältlichen halogenierten Pyridinderivate sollen dabei vorzugsweise mit guter Ausbeute, hoher Reinheit und in ökonomischer Weise erhalten werden.

Überraschenderweise wurde gefunden, dass in einem Verfahren unter Verwendung einer Zink-metallorganischen Base halogenierte Pyridinderivate der Formel (II) vorteilhaft hergestellt werden können.

Gegenstand der vorliegenden Erfindung ist demnach ein Verfahren zur Herstellung von Verbindungen der Formel (II) 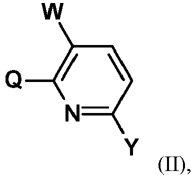 in welcher (Ausgestaltung 1)
- Q: für ein Strukturelement 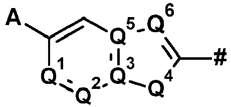 steht, wobei das Zeichen # die Bindung zum Rest des Moleküls anzeigt und
Q¹ für N oder CR⁶ steht,
Q² für N oder CR⁶ steht,
Q³ für N oder C steht,
Q⁴ für O, S, N oder NR⁷ steht,
Q⁵ für N oder C steht,
Q⁶ für N oder CH steht,
R⁶ für Wasserstoff, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht,
R⁷ für (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht, und
A für Wasserstoff, Cyano, Halogen, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl oder Di-(C₁-C₄)alkylaminosulfonylsteht steht,
oder A für -O-CF₂-O- steht und gemeinsam mit Q¹ und dem Kohlenstoffatom, an das es gebunden ist, einen fünfgliedrigen Ring bildet, wobei Q¹ für Kohlenstoff steht,
- W: für Halogen steht, und
- Y: für Halogen, CO₂R¹ oder NO₂ steht, wobei R¹ für (C₁-C₆)-Alkyl oder (C₁-C₆)-Haloalkyl steht,
dadurch gekennzeichnet, dass in einem ersten Verfahrensschritt a) eine Verbindung Q-H, in welcher Q die oben genannte Bedeutung hat,
mit einer Zink-metallorganischen Base der Struktur (NR³R⁴)-Zn-R² oder (NR³R⁴)₂-Zn, in welcher
R² für Halogen oder -O-Pivaloyl steht und
R³ und R⁴ gemeinsam eine -(CH₂)₄-, -(CH₂)₅- oder -(CH₂)₂O(CH₂)₂-Gruppe bilden, wobei jede dieser Gruppen optional durch 1, 2, 3 oder 4 R⁵-Radikale substituiert sein kann und R⁵ ausgewählt ist aus der Gruppe bestehend aus Methyl, Ethyl, n-Propyl und i-Propyl,
zu einer Verbindung der Formel (IIIa) oder der Formel (IIIb) umgesetzt wird,

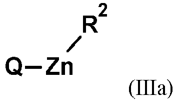

**Q-Zn-Q** (IIIb),

in welcher Q und R² jeweils die oben genannten Bedeutungen haben,
und diese Verbindung der Formel (IIIa) oder (IIIb) in einem zweiten Verfahrensschritt b) mit einer Verbindung der Formel (I) 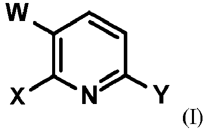 in welcher X für Halogen steht und W und Y jeweils die oben genannten Bedeutungen haben, in Gegenwart eines Katalysators zur Verbindung der Formel (II) umgesetzt wird.

Vorzugsweise stehen Q¹, Q², Q³, Q⁴, Q⁵ und Q⁶ für nicht mehr als insgesamt fünf Stickstoffatome und weiter bevorzugt für nicht mehr als insgesamt vier Stickstoffatome.

Bevorzugte und besonders bevorzugte Bedeutungen der in den vorstehend erwähnten Formeln (I), (II) (IIIa) und (IIIb) des erfindungsgemäßen Verfahrens aufgeführten Reste Q, W, R¹, R², X, und Y werden im Folgenden erläutert, wobei die Zink-metallorganische Base weiter unten noch genau beschrieben wird, so dass die bevorzugten Ausgestaltungen der Base dort angegeben sind.
(Ausgestaltung 2)
   - Q: steht bevorzugt für ein Strukturelement aus der Reihe Q1 bis Q15, 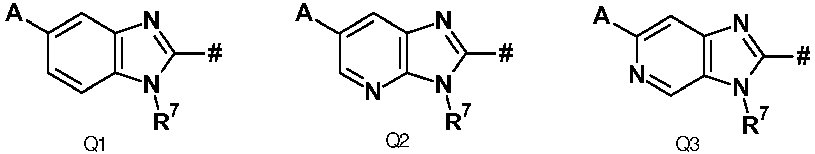 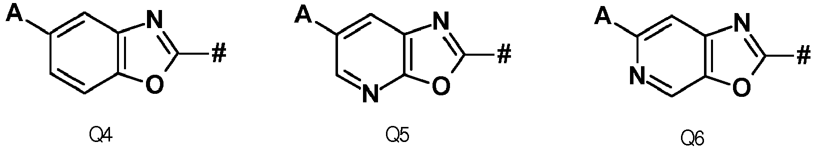 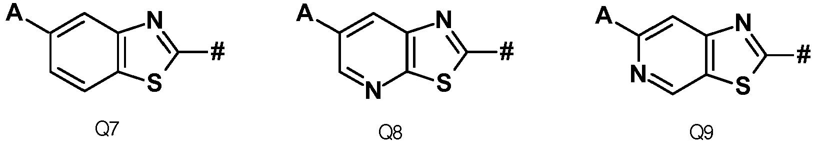 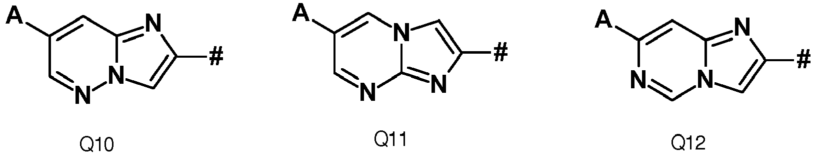 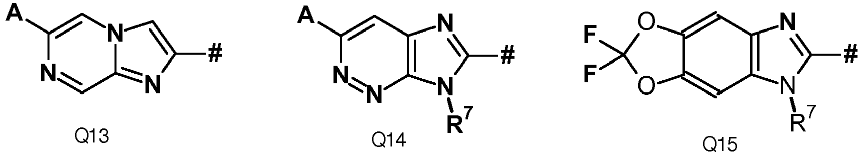
   - R⁷: steht bevorzugt für (C₁-C₄)Alkyl, (C₁-C₄)Halogenalkyl, (C₁-C₄)Alkoxy-(C₁-C₄)alkyl, (C₁-C₄)Halogenalkoxy-(C₁-C₄)alkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl,
   - A: steht bevorzugt für Fluor, Chlor, Brom, Fluormethyl, Difluormethyl, Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethoxy, Difluorchlormethoxy, Dichlorfluormethoxy, Trifluormethylthio, Trifluormethylsulfinyl oder Trifluormethylsulfonyl,
   - W: steht bevorzugt für Fluor, Chlor oder Brom,
   - R²: steht bevorzugt für Halogen, insbesondere Chlor, Brom oder Iod,
   - X: steht bevorzugt für Halogen, insbesondere Brom oder Iod, und
   - Y: steht bevorzugt für Fluor, Chlor, Brom, CO₂R¹ oder NO₂, wobei R¹ für (C₁-C₄)-Alkyl steht.
(Ausgestaltung 3)
   - Q: steht besonders bevorzugt für ein Strukturelement aus der Reihe Q2, Q3, Q10, Q12, Q14 oder Q15,
   - R⁷: steht besonders bevorzugt für (C₁-C₄)Alkyl oder (C₁-C₄)Alkoxy-(C₁-C₄)alkyl,
   - A: steht besonders bevorzugt für Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethylthio, Trifluormethylsulfinyl oder Trifluormethylsulfonyl,
   - W: steht besonders bevorzugt für Fluor oder Chlor, insbesondere Fluor,
   - R²: steht besonders bevorzugt für Chlor,
   - X: steht besonders bevorzugt für Brom oder Iod, insbesondere Iod, und
   - Y: steht besonders bevorzugt für Chlor, Brom, CO₂R¹ oder NO₂, wobei R¹ für (C₁-C₄)-Alkyl steht.
(Ausgestaltung 4)
   - Q: steht ganz besonders bevorzugt für das Strukturelement Q3 oder Q14,
   - R⁷: steht ganz besonders bevorzugt für Methyl, Ethyl, n-Propyl oder iso-Propyl, insbesondere Methyl,
   - A: steht ganz besonders bevorzugt für Trifluormethyl,
   - W: steht ganz besonders bevorzugt für Fluor,
   - R²: steht ganz besonders bevorzugt für Chlor,
   - X: steht ganz besonders bevorzugt für Iod, und
   - Y: steht ganz besonders bevorzugt für Chlor, Brom, CO₂R¹ oder NO₂, wobei R¹ für Methyl steht.

Die vorstehend aufgeführten Restedefinitionen bzw. Erläuterungen gelten sowohl für die Endprodukte und Zwischenprodukte als auch für die Ausgangsprodukte entsprechend. Diese Restedefinitionen können untereinander, also auch zwischen den jeweiligen Vorzugsbereichen, beliebig kombiniert werden.

Erfindungsgemäß bevorzugt werden solche Verbindungen, in welchen eine Kombination der vorstehend als bevorzugt aufgeführten Bedeutungen vorliegt.

Erfindungsgemäß besonders bevorzugt werden solche Verbindungen, in welchen eine Kombination der vorstehend als besonders bevorzugt aufgeführten Bedeutungen vorliegt.

Erfindungsgemäß ganz besonders bevorzugt werden solche Verbindungen, in welchen eine Kombination der vorstehend als ganz besonders bevorzugt aufgeführten Bedeutungen vorliegt.

In einer weiteren bevorzugten Ausführungsform der Erfindung steht Q für Q1 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 5).

In einer weiteren bevorzugten Ausführungsform der Erfindung steht Q für Q2 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 6).

In einer weiteren bevorzugten Ausführungsform der Erfindung steht Q für Q3 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 7).

In einer weiteren bevorzugten Ausführungsform der Erfindung steht Q für Q4 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 8).

In einer weiteren bevorzugten Ausführungsform der Erfindung steht Q für Q5 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 9).

In einer weiteren bevorzugten Ausführungsform der Erfindung steht Q für Q6 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 10).

In einer weiteren bevorzugten Ausführungsform der Erfindung steht Q für Q7 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 11).

In einer weiteren bevorzugten Ausführungsform der Erfindung steht Q für Q8 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 12).

In einer weiteren bevorzugten Ausführungsform der Erfindung steht Q für Q9 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 13).

In einer weiteren bevorzugten Ausführungsform der Erfindung steht Q für Q10 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 14).

In einer weiteren bevorzugten Ausführungsform der Erfindung steht Q für Q11 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 15).

In einer weiteren bevorzugten Ausführungsform der Erfindung steht Q für Q12 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 16).

In einer weiteren bevorzugten Ausführungsform der Erfindung steht Q für Q13 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 17).

In einer weiteren bevorzugten Ausführungsform der Erfindung steht Q für Q14 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 18).

In einer weiteren bevorzugten Ausführungsform der Erfindung steht Q für Q15 und R⁷, A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 19).

Vorteilhafterweise lassen sich die halogenierten Pyridinderivate der Formel (II) mit dem erfindungsgemäßen Verfahren mit guten Ausbeuten und in hoher Reinheit herstellen. Ein großer Vorteil des erfindungsgemäßen Verfahrens ist dessen Regioselektivität. Aufgrund der sehr guten funktionellen Gruppen-Toleranz von Zink-Reagenzien sind Zinkbasen sehr attraktiv. Insbesondere vorteilhaft ist ferner die Möglichkeit, auch bei deutlich niedrigeren Temperaturen Negishi-Kupplungen durchführen zu können, wobei in erfindungsgemäßen Verfahren auch bei höheren Temperaturen empfindliche funktionelle Gruppen wie Ester oder Fluoratome toleriert werden, ohne die die vorhandene Regioselektivität zu beeinträchtigen. Ferner können Negishi Kreuzkupplungen im Rahmen eines erfindungsgemäßen Verfahrens auch in Gegenwart von ortho-Substituenten am Pyridingerüst gute Ausbeuten an Zielprodukt ergeben, obwohl solche Kupplungen mit 2-substituierten Pyridinderivaten bislang dafür bekannt sind, niedrige Ausbeuten zu liefern. Somit werden weitere und/oder flexiblere Derivatisierungen von Edukt und Produkt möglich, ohne Synthesenrouten ständig ändern bzw. anzupassen zu müssen.

Das erfindungsgemäße Verfahren kann anhand des folgenden Schemas (I) erläutert werden:

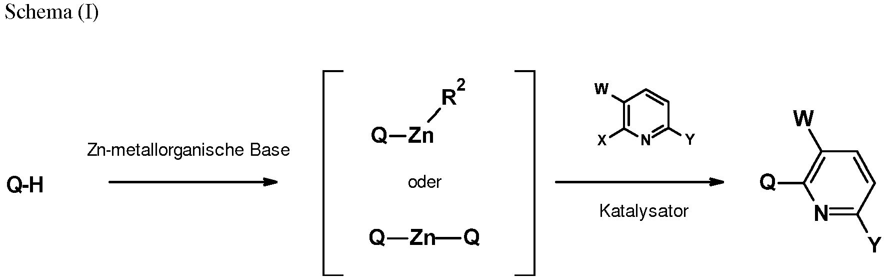

Hierin haben Q, W, R², X, und Y sowie die innerhalb der jeweiligen Definitionen ggfs. vorhandenen weiteren Strukturelemente jeweils die vorstehend angegebenen Bedeutungen. Die in Klammern angegebenen Verbindungen stellen die Zwischenstufe (Formel IIIa beziehungsweise IIIb) dar, welche mit einer Verbindung der Formel (I) weiter zur Verbindung der Formel (II) umgesetzt wird. Demnach lässt sich das erfindungsgemäße Verfahren in die beiden Verfahrensschritte a) und b) unterteilen, wobei Schritt a) die Umsetzung der Verbindung Q-H zur jeweiligen Zwischenstufe und Schritt b) die weitere Umsetzung der Zwischenstufe zur Verbindung der Formel (II) ist.

### Allgemeine Definitionen

Im Zusammenhang mit der vorliegenden Erfindung umfasst der Begriff Halogen (Hal), soweit nicht anders definiert, solche Elemente, die ausgewählt sind aus der Gruppe bestehend aus Fluor, Chlor, Brom und Iod.

Der Begriff "Halogenide" beschreibt im Zusammenhang mit der vorliegenden Erfindung Verbindungen zwischen Halogenen und Elementen anderer Gruppen des Periodensystems, wobei salzartige Halogenide (ionische Verbindungen (Salze), die aufgrund der großen Elektronegativitätsdifferenz zwischen den beteiligten Elementen aus Anionen und Kationen bestehen und durch elektrostatische Wechselwirkungen zusammengehalten werden) oder kovalente Halogenide (kovalente Verbindungen, bei denen der Elektronegativitätsunterschied nicht so groß ist wie bei den vorstehend genannten ionischen Verbindungen, die Bindungen jedoch eine Ladungspolarität aufweisen) vorliegen können, abhängig von der Art der chemischen Bindung. Erfindungsgemäß besonders bevorzugt sind salzartige Halogenide.

Der Begriff "Pivaloyl" beschreibt im Zusammenhang mit der vorliegenden Erfindung den deprotonierten Rest der Pivalinsäure (X) mit der Summenformel (CH₃)₃CCO₂H. 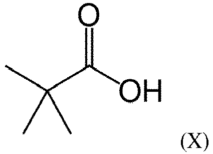 "O-Pivaloyl" bedeutet entsprechend, dass die Bindung des Pivaloylrestes über das deprotonierte Sauerstoffatom der Säuregruppe erfolgt.

Sofern nicht an anderer Stelle anders definiert, wird unter dem Begriff "Alkyl", entweder in Alleinstellung oder aber in Kombination mit weiteren Begriffen, wie beispielsweise Halogenalkyl, im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für C₁-C₁₂-Alkylreste sind Methyl, Ethyl, n-Propyl, iso-Propyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl n-Heptyl, n-Octyl, n-Nonyl, n-Decyl, n-Undecyl und n-Dodecyl. Von diesen Alkylresten sind C₁-C₆-Alkylreste besonders bevorzugt. Insbesondere bevorzugt sind C₁-C₄-Alkylreste.

Sofern nicht an anderer Stelle anders definiert, wird unter dem Begriff "Alkenyl", entweder in Alleinstellung oder aber in Kombination mit weiteren Begriffen, erfindungsgemäß ein linearer oder verzweigter C₂-C₁₂-Alkenylrest, welcher mindestens eine Doppelbindung aufweist, beispielsweise Vinyl, Allyl, 1-Propenyl, Isopropenyl, 1-Butenyl, 2-Butenyl, 3-Butenyl, 1,3-Butadienyl, 1-Pentenyl, 2-Pentenyl, 3-Pentenyl, 4-Pentenyl, 1,3-Pentadienyl, 1-Hexenyl, 2-Hexenyl, 3-Hexenyl, 4-Hexenyl, 5-Hexenyl und 1,4-Hexadienyl, verstanden. Bevorzugt hiervon sind C₂-C₆-Alkenylreste und besonders bevorzugt sind C₂-C₄-Alkenylreste.

Sofern nicht an anderer Stelle anders definiert, wird unter dem Begriff "Alkinyl", entweder in Alleinstellung oder aber in Kombination mit weiteren Begriffen, erfindungsgemäß ein linearer oder verzweigter C₂-C₁₂-Alkinylrest, welcher mindestens eine Dreifachbindung aufweist, beispielsweise Ethinyl, 1-Propinyl und Propargyl, verstanden. Bevorzugt hiervon sind C₃-C₆-Alkinylreste und besonders bevorzugt sind C₃-C₄-Alkinylreste. Der Alkinylrest kann dabei auch mindestens eine Doppelbindung aufweisen.

Sofern nicht an anderer Stelle anders definiert, wird unter dem Begriff "Cycloalkyl", entweder in Alleinstellung oder aber in Kombination mit weiteren Begriffen, erfindungsgemäß ein C₃-C₈-Cycloalkylrest verstanden, beispielsweise Cyclopropyl, Cyclobutyl, Cyclopentyl, Cyclohexyl, Cycloheptyl und Cyclooctyl, verstanden. Bevorzugt hiervon sind C₃-C₆-Cycloalkylreste.

Unter dem Begriff "Alkoxy", entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die oben stehende Bedeutung aufweist.

Durch Halogen substituierte Reste, z.B. Halogenalkyl (=Haloalkyl), sind einfach oder mehrfach bis zur maximal möglichen Substituentenzahl halogeniert. Bei mehrfacher Halogenierung können die Halogenatome gleich oder verschieden sein. Soweit nicht anders definiert steht Halogen dabei für Fluor, Chlor, Brom oder Iod, insbesondere für Fluor, Chlor oder Brom. Mit einem oder mehreren Halogenatomen (-Hal) substituierte Alkyl-Gruppen sind beispielsweise ausgewählt aus Trifluormethyl (CF₃), Difluormethyl (CHF₂), CF₃CH₂, ClCH₂ oder CF₃CCl₂.

Gegebenenfalls substituierte Reste können, wenn nichts anderes erwähnt ist, einfach oder mehrfach substituiert sein, wobei bei Mehrfachsubstitutionen die Substituenten gleich oder verschieden sein können.

Die Synthese von Verbindungen Q-H als Edukte eines erfindungsgemäßen Verfahrens ist dem Fachmann grundsätzlich bekannt. Beispielsweise können Verbindungen Q-H mit Q = Q1, Q2, Q3, Q14 oder Q15 aus entsprechenden Pyridin-Diaminderivaten durch Ringschluss zur jeweiligen Azolverbindung erhalten werden, wie zum Beispiel in WO2014/100065 oder WO2015/017610 beschrieben, vorzugsweise unter sauren Bedingungen. Alternativsynthesen sind ebenfalls möglich, sind jedoch komplexer und dadurch im Regelfall wirtschaftlich nachteiliger.

Die Umsetzung der Verbindungen Q-H zu Verbindungen der Formel (IIIa) oder Formel (IIIb) im ersten Verfahrensschritt (Schritt a)) erfolgt in Gegenwart einer Zink-metallorganischen Base der Struktur (NR³R⁴)-Zn-R² oder (NR³R⁴)₂-Zn, in welcher (Ausgestaltung B-1)
R² wie vorstehend (Ausgestaltung 1) definiert ist (daher für Halogen oder -O-Pivaloyl steht),
R³ und R⁴ gemeinsam eine -(CH₂)₄-, -(CH₂)₅- oder -(CH₂)₂O(CH₂)₂-Gruppe bilden, wobei jede dieser Gruppen optional durch 1, 2, 3 oder 4 R⁵-Radikale substituiert sein kann und
R⁵ ausgewählt ist aus der Gruppe bestehend aus Methyl, Ethyl, n-Propyl und i-Propyl.

Bevorzugt ist, dass (Ausgestaltung B-2)
R² wie vorstehend als bevorzugt (Ausgestaltung 2) definiert ist (daher für Halogen steht, insbesondere für Chlor, Brom oder Iod),
R³ und R⁴ gemeinsam eine -(CH₂)₅-Gruppe bilden, wobei jede dieser Gruppen optional durch 1, 2, 3 oder 4 R⁵-Radikale substituiert sein kann und
R⁵ ausgewählt ist aus der Gruppe bestehend aus Methyl und Ethyl.

Besonders bevorzugt ist, dass (Ausgestaltung B-3)
R² wie vorstehend als besonders bevorzugt (Ausgestaltung 3) oder als ganz besonders bevorzugt (Ausgestaltung 4) definiert ist (daher für Chlor steht) und
R³ und R⁴ gemeinsam eine -(CH₂)₅-Gruppe bilden, die durch 4 Methylgruppen substituiert ist.

Die vorstehend aufgeführten Restedefinitionen können untereinander, also auch zwischen den jeweiligen Vorzugsbereichen, beliebig kombiniert werden.

In einer ganz besonders bevorzugten Ausgestaltung der erfindungsgemäßen Base ist das Strukturelement (NR³R⁴) Tetramethylpiperidin (TMP) gemäß Formel (IV).

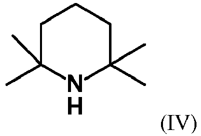

Erfindungsgemäß ganz besonders bevorzugte Zink-metallorganische Basen sind demnach dadurch gekennzeichnet, dass Zink gebunden an TMP vorliegt, insbesondere als Zinkhalogenid und ganz besonders bevorzugt als Zinkchlorid. Derartige Basen weisen folgende Struktur der Formel (V) auf (Ausgestaltung B-4)

(V) (TMP)ₓZnCl₂₋ₓ,

worin x für die Zahl 1 oder 2 steht. Hierunter wiederum bevorzugt sind Basen mit x=1 (Ausgestaltung B-5) gemäß Formel (VI):

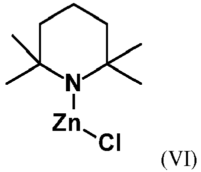

In einer weiteren bevorzugten Ausführungsform des erfindungsgemäßen Verfahrens liegt die Zink-metallorganische Base in Verbindung mit Alkali- oder Erdalkalihalogeniden vor. Dies gilt insbesondere für Basen der Formeln (V) und (VI). Besonders bevorzugte derartige Alkali- oder Erdalkalihalogenide sind Lithiumchlorid und Magnesiumchlorid, ganz besonders bevorzugt ist Lithiumchlorid. Erfindungsgemäß ganz besonders bevorzugte Zink-metallorganische Basen sind demnach TMP ZnCl·LiCl oder (TMP)₂ Zn·2LiCl (Ausgestaltung B-6). Am meisten bevorzugt ist TMP ZnCl·LiCl (VII; Ausgestaltung B-7).

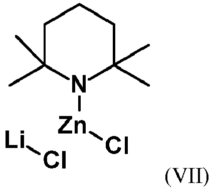

Beispielhaft werden in nachfolgender Tabelle 1 konkrete Kombinationen von Verbindungen der Formeln (I), (II) und (IIIa) bzw. (IIIb) mit erfindungsgemäßen Basen genannt, die in einem erfindungsgemäßen Verfahren zur Anwendung kommen können. Da in einigen Ausgestaltungen das Strukturelement R² sowohl in der erfindungsgemäßen Base als auch in der Verbindung der Formel (IIIa) vorliegt, gilt für R² jeweils die engste Definition.

**Tabelle 1:**

| Nummer | Verbindungen der Formeln (I), (II) und (IIIa) bzw. (IIIb) | Base gemäß |
|---|---|---|
| 1 | Ausgestaltung 1 | Ausgestaltung B-1 |
| 2 | Ausgestaltung 1 | Ausgestaltung B-2 |
| 3 | Ausgestaltung 1 | Ausgestaltung B-3 |
| 4 | Ausgestaltung 1 | Ausgestaltung B-4 |
| 5 | Ausgestaltung 1 | Ausgestaltung B-5 |
| 6 | Ausgestaltung 1 | Ausgestaltung B-6 |
| 7 | Ausgestaltung 1 | Ausgestaltung B-7 |
| 8 | Ausgestaltung 2 | Ausgestaltung B-1 |
| 9 | Ausgestaltung 2 | Ausgestaltung B-2 |
| 10 | Ausgestaltung 2 | Ausgestaltung B-3 |
| 11 | Ausgestaltung 2 | Ausgestaltung B-4 |
| 12 | Ausgestaltung 2 | Ausgestaltung B-5 |
| 13 | Ausgestaltung 2 | Ausgestaltung B-6 |
| 14 | Ausgestaltung 2 | Ausgestaltung B-7 |
| 15 | Ausgestaltung 3 | Ausgestaltung B-1 |
| 16 | Ausgestaltung 3 | Ausgestaltung B-2 |
| 17 | Ausgestaltung 3 | Ausgestaltung B-3 |
| 18 | Ausgestaltung 3 | Ausgestaltung B-4 |
| 19 | Ausgestaltung 3 | Ausgestaltung B-5 |
| 20 | Ausgestaltung 3 | Ausgestaltung B-6 |
| 21 | Ausgestaltung 3 | Ausgestaltung B-7 |
| 22 | Ausgestaltung 4 | Ausgestaltung B-1 |
| 23 | Ausgestaltung 4 | Ausgestaltung B-2 |
| 24 | Ausgestaltung 4 | Ausgestaltung B-3 |
| 25 | Ausgestaltung 4 | Ausgestaltung B-4 |
| 26 | Ausgestaltung 4 | Ausgestaltung B-5 |
| 27 | Ausgestaltung 4 | Ausgestaltung B-6 |
| 28 | Ausgestaltung 4 | Ausgestaltung B-7 |
| 29 | Ausgestaltung 5 | Ausgestaltung B-1 |
| 30 | Ausgestaltung 5 | Ausgestaltung B-2 |
| 31 | Ausgestaltung 5 | Ausgestaltung B-3 |
| 32 | Ausgestaltung 5 | Ausgestaltung B-4 |
| 33 | Ausgestaltung 5 | Ausgestaltung B-5 |
| 34 | Ausgestaltung 5 | Ausgestaltung B-6 |
| 35 | Ausgestaltung 5 | Ausgestaltung B-7 |
| 36 | Ausgestaltung 6 | Ausgestaltung B-1 |
| 37 | Ausgestaltung 6 | Ausgestaltung B-2 |
| 38 | Ausgestaltung 6 | Ausgestaltung B-3 |
| 39 | Ausgestaltung 6 | Ausgestaltung B-4 |
| 40 | Ausgestaltung 6 | Ausgestaltung B-5 |
| 41 | Ausgestaltung 6 | Ausgestaltung B-6 |
| 42 | Ausgestaltung 6 | Ausgestaltung B-7 |
| 43 | Ausgestaltung 7 | Ausgestaltung B-1 |
| 44 | Ausgestaltung 7 | Ausgestaltung B-2 |
| 45 | Ausgestaltung 7 | Ausgestaltung B-3 |
| 46 | Ausgestaltung 7 | Ausgestaltung B-4 |
| 47 | Ausgestaltung 7 | Ausgestaltung B-5 |
| 48 | Ausgestaltung 7 | Ausgestaltung B-6 |
| 49 | Ausgestaltung 7 | Ausgestaltung B-7 |
| 50 | Ausgestaltung 8 | Ausgestaltung B-1 |
| 51 | Ausgestaltung 8 | Ausgestaltung B-2 |
| 52 | Ausgestaltung 8 | Ausgestaltung B-3 |
| 53 | Ausgestaltung 8 | Ausgestaltung B-4 |
| 54 | Ausgestaltung 8 | Ausgestaltung B-5 |
| 55 | Ausgestaltung 8 | Ausgestaltung B-6 |
| 56 | Ausgestaltung 8 | Ausgestaltung B-7 |
| 57 | Ausgestaltung 9 | Ausgestaltung B-1 |
| 58 | Ausgestaltung 9 | Ausgestaltung B-2 |
| 59 | Ausgestaltung 9 | Ausgestaltung B-3 |
| 60 | Ausgestaltung 9 | Ausgestaltung B-4 |
| 61 | Ausgestaltung 9 | Ausgestaltung B-5 |
| 62 | Ausgestaltung 9 | Ausgestaltung B-6 |
| 63 | Ausgestaltung 9 | Ausgestaltung B-7 |
| 64 | Ausgestaltung 10 | Ausgestaltung B-1 |
| 65 | Ausgestaltung 10 | Ausgestaltung B-2 |
| 66 | Ausgestaltung 10 | Ausgestaltung B-3 |
| 67 | Ausgestaltung 10 | Ausgestaltung B-4 |
| 68 | Ausgestaltung 10 | Ausgestaltung B-5 |
| 69 | Ausgestaltung 10 | Ausgestaltung B-6 |
| 70 | Ausgestaltung 10 | Ausgestaltung B-7 |
| 71 | Ausgestaltung 11 | Ausgestaltung B-1 |
| 72 | Ausgestaltung 11 | Ausgestaltung B-2 |
| 73 | Ausgestaltung 11 | Ausgestaltung B-3 |
| 74 | Ausgestaltung 11 | Ausgestaltung B-4 |
| 75 | Ausgestaltung 11 | Ausgestaltung B-5 |
| 76 | Ausgestaltung 11 | Ausgestaltung B-6 |
| 77 | Ausgestaltung 11 | Ausgestaltung B-7 |
| 78 | Ausgestaltung 12 | Ausgestaltung B-1 |
| 79 | Ausgestaltung 12 | Ausgestaltung B-2 |
| 80 | Ausgestaltung 12 | Ausgestaltung B-3 |
| 81 | Ausgestaltung 12 | Ausgestaltung B-4 |
| 82 | Ausgestaltung 12 | Ausgestaltung B-5 |
| 83 | Ausgestaltung 12 | Ausgestaltung B-6 |
| 84 | Ausgestaltung 12 | Ausgestaltung B-7 |
| 85 | Ausgestaltung 13 | Ausgestaltung B-1 |
| 86 | Ausgestaltung 13 | Ausgestaltung B-2 |
| 87 | Ausgestaltung 13 | Ausgestaltung B-3 |
| 88 | Ausgestaltung 13 | Ausgestaltung B-4 |
| 89 | Ausgestaltung 13 | Ausgestaltung B-5 |
| 90 | Ausgestaltung 13 | Ausgestaltung B-6 |
| 91 | Ausgestaltung 13 | Ausgestaltung B-7 |
| 92 | Ausgestaltung 14 | Ausgestaltung B-1 |
| 93 | Ausgestaltung 14 | Ausgestaltung B-2 |
| 94 | Ausgestaltung 14 | Ausgestaltung B-3 |
| 95 | Ausgestaltung 14 | Ausgestaltung B-4 |
| 96 | Ausgestaltung 14 | Ausgestaltung B-5 |
| 97 | Ausgestaltung 14 | Ausgestaltung B-6 |
| 98 | Ausgestaltung 14 | Ausgestaltung B-7 |
| 99 | Ausgestaltung 15 | Ausgestaltung B-1 |
| 100 | Ausgestaltung 15 | Ausgestaltung B-2 |
| 101 | Ausgestaltung 15 | Ausgestaltung B-3 |
| 102 | Ausgestaltung 15 | Ausgestaltung B-4 |
| 103 | Ausgestaltung 15 | Ausgestaltung B-5 |
| 104 | Ausgestaltung 15 | Ausgestaltung B-6 |
| 105 | Ausgestaltung 15 | Ausgestaltung B-7 |
| 106 | Ausgestaltung 16 | Ausgestaltung B-1 |
| 107 | Ausgestaltung 16 | Ausgestaltung B-2 |
| 108 | Ausgestaltung 16 | Ausgestaltung B-3 |
| 109 | Ausgestaltung 16 | Ausgestaltung B-4 |
| 110 | Ausgestaltung 16 | Ausgestaltung B-5 |
| 111 | Ausgestaltung 16 | Ausgestaltung B-6 |
| 112 | Ausgestaltung 16 | Ausgestaltung B-7 |
| 113 | Ausgestaltung 17 | Ausgestaltung B-1 |
| 114 | Ausgestaltung 17 | Ausgestaltung B-2 |
| 115 | Ausgestaltung 17 | Ausgestaltung B-3 |
| 116 | Ausgestaltung 17 | Ausgestaltung B-4 |
| 117 | Ausgestaltung 17 | Ausgestaltung B-5 |
| 118 | Ausgestaltung 17 | Ausgestaltung B-6 |
| 119 | Ausgestaltung 17 | Ausgestaltung B-7 |
| 120 | Ausgestaltung 18 | Ausgestaltung B-1 |
| 121 | Ausgestaltung 18 | Ausgestaltung B-2 |
| 122 | Ausgestaltung 18 | Ausgestaltung B-3 |
| 123 | Ausgestaltung 18 | Ausgestaltung B-4 |
| 124 | Ausgestaltung 18 | Ausgestaltung B-5 |
| 125 | Ausgestaltung 18 | Ausgestaltung B-6 |
| 126 | Ausgestaltung 18 | Ausgestaltung B-7 |
| 127 | Ausgestaltung 19 | Ausgestaltung B-1 |
| 128 | Ausgestaltung 19 | Ausgestaltung B-2 |
| 129 | Ausgestaltung 19 | Ausgestaltung B-3 |
| 130 | Ausgestaltung 19 | Ausgestaltung B-4 |
| 131 | Ausgestaltung 19 | Ausgestaltung B-5 |
| 132 | Ausgestaltung 19 | Ausgestaltung B-6 |
| 133 | Ausgestaltung 19 | Ausgestaltung B-7 |

Vorzugsweise wird die Zink-metallorganische Base in dem erfindungsgemäßen Verfahren in einer Gesamtmenge von 0,5 bis 5 Äquivalenten, vorzugsweise von 0,8 bis 2 Äquivalenten, weiter bevorzugt von 1 bis 1,5 Äquivalenten und besonders bevorzugt von 1,0 bis 1,2 Äquivalenten, bezogen auf die Verbindung Q-H, eingesetzt. Ein Vorteil des erfindungsgemäßen Verfahrens ist es diesbezüglich, dass die metallorganische Base in nahezu stöchiometrischen Mengen eingesetzt werden kann.

In Abhängigkeit davon, ob in der eingesetzten Zink-metallorganischen Base das Strukturelement (NR³R⁴) einfach oder zweifach vorhanden ist, entstehen Zwischenverbindungen der Formel (IIIa) beziehungsweise der Formel (IIIb) in Verfahrensschritt a).

Die Umsetzung der Verbindungen der Formel (IIIa) beziehungsweise (IIIb) zu Verbindungen der Formel (II) im zweiten Verfahrensschritt (Schritt b)) erfolgt in Gegenwart einer Verbindung der Formel (I) 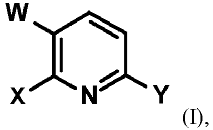 in welcher X, W und Y jeweils die oben genannten Bedeutungen haben.

Die Reaktion findet während der Negishi Kreuzkupplung nahezu ausschließlich an der 2. Position statt, da Iod die beste Abgangsgruppe an dem Pyridingerüst ist. Sie liefert dann regioselektiv das entsprechende Pyridinderivat der Formel (II).

Verbindungen der Formel (I) lassen sich beispielsweise durch Substitution einer entsprechenden Vorläuferverbindung, d.h. einem Pyridinderivat mit den Resten W und Y, mit dem Rest X erhalten. Eine derartige Substitution kann beispielsweise durch Metallierung der Vorläuferverbindung in Gegenwart von Zinkbasen und anschließender Umsetzung mit elentarem Halogen erfolgen. Derartige Metallierungen sind beispielsweise mit substituierten Pyridinen an der 4. Position in Angewandte Chemie 2007 (46), S. 7685ff oder Organic Letters 2009 (11), S. 1837ff beschrieben.

Vorzugsweise wird die Verbindung der Formel (I) in dem erfindungsgemäßen Verfahren in einer Gesamtmenge von 0,5 bis 10,0 Äquivalenten, vorzugsweise von 0,8 bis 5 Äquivalenten, weiter bevorzugt von 1 bis 2,5 Äquivalenten und besonders bevorzugt von 1,0 bis 1,5 Äquivalenten oder besonders bevorzugt von 1,5 bis 2,0 Äquivalenten oder besonders bevorzugt von 1,0 bis 2,0 Äquivalenten, bezogen auf die Verbindung Q-H, eingesetzt.

Die Umsetzung in Verfahrensschritt b) erfolgt ferner in Gegenwart eines Katalysators. Vorzugsweise ist der Katalysator eine Palladiumverbindung oder eine Nickelverbindung. Besonders bevorzugt ist der Katalysator eine Palladiumverbindung. Ganz besonders bevorzugt handelt es sich um Tetrakis(triphenylphosphine)palladium(0), abgekürzt Pd(PPh₃)₄, der Formel (IX).

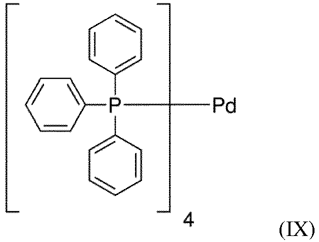

Üblicherweise werden in einem erfindungsgemäßen Verfahren 2,5-25 mol% und vorzugsweise 5-20 mol% Katalysator eingesetzt.

Die erfindungsgemäße Umsetzung der Verbindungen Q-H zu Verbindungen der Formel (IIIa) beziehungsweise (IIIb) und weiter zu Verbindungen der Formel (II) erfolgt vorzugsweise jeweils in Gegenwart eines organischen Lösungsmittels. Als Lösungsmittel kommen prinzipiell alle organischen Lösungsmittel in Frage, die unter den angewendeten Reaktionsbedingungen inert sind und in denen die umzusetzenden Verbindungen eine ausreichende Löslichkeit aufweisen. Als geeignete Lösungsmittel sind insbesondere zu nennen: Tetrahydrofuran (THF), 1,4-Dioxan, Diethylether, Diglyme, Methyltertbutylether (MTBE), tert-Amyl-methylether (TAME), 2-Methyl-THF, Toluol, Xylole, Mesitylen, Ethylencarbonat, Propylencarbonat, N,N-Dimethylacetamid, N,N-Dimethylformamid (DMF), N-Methylpyrrolidon (NMP), N-Ethyl-2-pyrrilidon (NEP), N-Butyl-2-pyrrilidon (NBP); N,N'-Dimethylpropylenharnstoff (DMPU), Halogenkohlenwasserstoffe und aromatische Kohlenwasserstoffe, insbesondere Chlorkohlenwasserstoffe wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, insbesondere 1,2-Dichlorbenzol, Chlortoluol, Trichlorbenzol; 4-Methoxybenzol, fluorierte Aliphate und Aromaten wie Trichlortrifluorethan, Benzotrifluorid und 4-Chlorbenzotrifluorid.Es können auch Lösungsmittelgemische, vorzugsweise Gemische aus den vorstehend genannten Lösungsmitteln wie Tetrahydrofuran (THF), 1,4-Dioxan, Diethylether, Diglyme, Methyltertbutylether (MTBE), tert-Amyl-methylether (TAME), 2-Methyl-THF, Toluol, Xylole, Mesitylen, Dimethylformamid (DMF), eingesetzt werden.

Bevorzugte Lösungsmittel sind THF, N,N-Dimethylformamid (DMF), 1,4-Dioxan, Diglyme, Methyltertbutylether (MTBE), tert-Amyl-methylether (TAME), 2-Methyl-THF, Toluol und 4-Methoxybenzol.

Besonders bevorzugte Lösungsmittel sind THF und N,N-Dimethylformamid (DMF), ganz besonders bevorzugt ist THF.

Das Lösungsmittel kann ferner entgast (sauerstoff-frei) sein.

Vorzugsweise wird für beide Verfahrensschritte a) und b) das gleiche Lösungsmittel verwendet. Alternative erfindungsgemäße Ausgestaltungen, in denen für die Verfahrensschritte a) und b) unterschiedliche Lösungsmittel verwendet werden, sind allerdings ebenfalls möglich, wobei die Lösungsmittel dann ebenfalls vorzugsweise aus den vorstehend genannten Lösungsmitteln ausgewählt sind und die jeweiligen als bevorzugt, besonders bevorzugt und ganz besonders bevorzugt angegebenen Lösungsmittel auf den jeweiligen Verfahrensschritt a) oder b) zu beziehen sind.

Die Umsetzung in Verfahrensschritt a) wird im Allgemeinen bei einer Temperatur zwischen 0°C und 80°C und zunehmend bevorzugt zwischen 10°C und 70°C, zwischen 15°C und 60°C, zwischen 20°C und 50°C, zwischen 20°C und 40°C und ganz besonders bevorzugt zwischen 20°C und 35°C, beispielsweise bei Raumtemperatur bzw. 25°C, durchgeführt.

Die Umsetzung in Verfahrensschritt b) wird im Allgemeinen bei einer Temperatur zwischen 40°C und 90°C und zunehmend bevorzugt zwischen 50°C und 85°C, zwischen 55°C und 80°C, zwischen 60°C und 80°C und ganz besonders bevorzugt zwischen 65°C und 75°C, beispielsweise bei 65°C, durchgeführt.

Die Reaktion wird üblicherweise bei Normaldruck durchgeführt, kann aber auch bei erhöhtem bzw. vermindertem Druck durchgeführt werden.

Die Isolierung der gewünschten Verbindungen der Formel (II) kann beispielsweise durch wässrige Aufarbeitung in Gegenwart von gesättigten Ammoniumchlorid- oder Natriumthiosulfat-Lösungen und/oder anschließende Chromatographie erfolgen. Solche Verfahren sind dem Fachmann bekannt und schließen auch eine Kristallisation aus einem organischen Lösungsmittel oder Lösungsmittelgemisch ein.

Ein Beispiel einer besonders bevorzugten Ausführungsform des erfindungsgemäßen Verfahrens kann anhand des folgenden Schemas (II) erläutert werden:

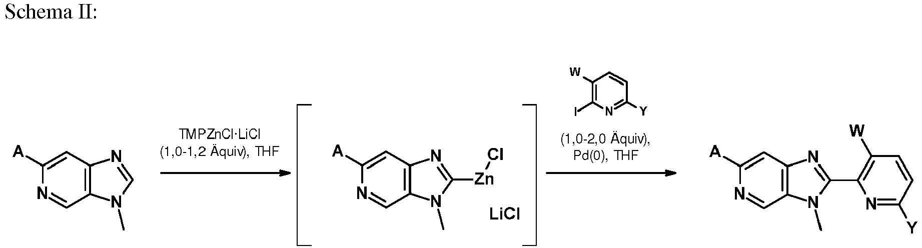

Hierin haben A, W und Y die vorstehend angegebenen Bedeutungen. Die in Klammern angegebene Verbindung stellt die entsprechende Zwischenstufe der Formel IIIa dar, welche weiter zum Produkt, einer Verbindung der Formel (II), umgesetzt wird. Beide Umsetzungen finden in THF als Lösungsmittel statt. "Äquiv" bezeichnet die eingesetzte Menge an Äquivalenten TMPZnCl·LiCl bzw. Verbindung der Formel (I). Pd(0) steht für eine Palladiumverbindung als Katalysator, vorzugsweise Pd(PPh₃)₄.

Ein weiterer Gegenstand der vorliegenden Erfindung sind Verbindungen der Struktur (Ausgestaltung Q-H-1-1) 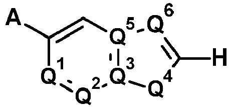 wobei
- Q¹: für N oder CR⁶ steht,
- Q²: für N oder CR⁶ steht,
- Q³: für N oder C steht,
- Q⁴: für O, S, N oder NR⁷ steht,
- Q⁵: für N oder C steht,
- Q⁶: für N oder CH steht,
- R⁶: für Wasserstoff, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht,
- R⁷: für (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht, und
- A: für Wasserstoff, Cyano, Halogen, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl oder Di-(C₁-C₄)alkylaminosulfonylsteht steht,
oder A für -O-CF₂-O- steht und gemeinsam mit Q¹ und dem Kohlenstoffatom, an das es gebunden ist, einen fünfgliedrigen Ring bildet, wobei Q¹ für Kohlenstoff steht.

Eine alternative Ausführungsform sind Verbindungen der Struktur (Ausgestaltung Q-H-1-2) 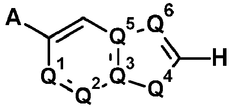 wobei
- Q¹: für N oder CR⁶ steht,
- Q²: für N oder CR⁶ steht,
- Q³: für N oder C steht,
- Q⁴: für O, S, N oder NR⁷ steht,
- Q⁵: für N oder C steht,
- Q⁶: für N oder CH steht,
- R⁶: für Wasserstoff, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht,
- R⁷: für (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht, und
- A: für Cyano, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl oder Di-(C₁-C₄)alkylaminosulfonylsteht steht,
oder A für -O-CF₂-O- steht und gemeinsam mit Q¹ und dem Kohlenstoffatom, an das es gebunden ist, einen fünfgliedrigen Ring bildet, wobei Q¹ für Kohlenstoff steht.

Vorzugsweise stehen Q¹, Q², Q³, Q⁴, Q⁵ und Q⁶ in Ausgestaltung Q-H-1-1 und Ausgestaltung Q-H-1-2 für nicht mehr als insgesamt fünf Stickstoffatome und weiter bevorzugt für nicht mehr als insgesamt vier Stickstoffatome.

Bevorzugte (Ausgestaltung Q-H-2-1 und Q-H-2-2), besonders bevorzugte (Ausgestaltung Q-H-3-1) und ganz besonders bevorzugte (Ausgestaltung Q-H-4-1) Bedeutungen der in den vorstehenden Ausgestaltungen Q-H-1-1 und Q-H-1-2 erwähnten Verbindungen Q-H aufgeführten Reste werden im Folgenden erläutert.
Ausgestaltung Q-H-2-1:
   - Q: steht bevorzugt für ein Strukturelement aus der Reihe Q1 bis Q15,
   - R⁷: steht bevorzugt für (C₁-C₄)Alkyl, (C₁-C₄)Halogenalkyl, (C₁-C₄)Alkoxy-(C₁-C₄)alkyl, (C₁-C₄)Halogenalkoxy-(C₁-C₄)alkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl, und
   - A: steht bevorzugt für Fluor, Chlor, Brom, Fluormethyl, Difluormethyl, Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethoxy, Difluorchlormethoxy, Dichlorfluormethoxy, Trifluormethylthio, Trifluormethylsulfinyl oder Trifluormethylsulfonyl.
Ausgestaltung Q-H-2-2:
   - Q: steht bevorzugt für ein Strukturelement aus der Reihe Q1 bis Q15,
   - R⁷: steht bevorzugt für (C₁-C₄)Alkyl, (C₁-C₄)Halogenalkyl, (C₁-C₄)Alkoxy-(C₁-C₄)alkyl, (C₁-C₄)Halogenalkoxy-(C₁-C₄)alkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl, und
   - A: steht bevorzugt für Fluormethyl, Difluormethyl, Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethoxy, Difluorchlormethoxy, Dichlorfluormethoxy, Trifluormethylthio, Trifluormethylsulfinyl oder Trifluormethylsulfonyl.
Ausgestaltung Q-H-3-1:
   - Q: steht besonders bevorzugt für ein Strukturelement aus der Reihe Q2, Q3, Q10, Q12, Q14 oder Q15,
   - R⁷: steht besonders bevorzugt für (C₁-C₄)Alkyl oder (C₁-C₄)Alkyoxy-(C₁-C₄)alkyl, und
   - A: steht besonders bevorzugt für Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethylthio, Trifluormethylsulfinyl oder Trifluormethylsulfonyl.
Ausgestaltung Q-H-4-1:
   - Q: steht ganz besonders bevorzugt für das Strukturelement Q3 oder Q14,
   - R⁷: steht ganz besonders bevorzugt für Methyl, Ethyl, n-Propyl oder iso-Propyl, insbesondere Methyl, und
   - A: steht ganz besonders bevorzugt für Trifluormethyl.

Die vorstehend aufgeführten Restedefinitionen können untereinander, also auch zwischen den jeweiligen Vorzugsbereichen, beliebig kombiniert werden.

Erfindungsgemäß bevorzugt werden solche Verbindungen, in welchen eine Kombination der vorstehend als bevorzugt aufgeführten Bedeutungen vorliegt.

Erfindungsgemäß besonders bevorzugt werden solche Verbindungen, in welchen eine Kombination der vorstehend als besonders bevorzugt aufgeführten Bedeutungen vorliegt.

Erfindungsgemäß ganz besonders bevorzugt werden solche Verbindungen, in welchen eine Kombination der vorstehend als ganz besonders bevorzugt aufgeführten Bedeutungen vorliegt.

Ein Beispiel für eine derartige ganz besonders bevorzugte Verbindung ist:
Q-X-1: 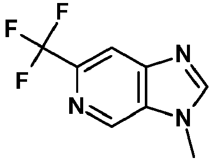 3-Methyl-6-(trifluormethyl)-3H-imidazo[4,5-c]pyridin
Q-X-2: 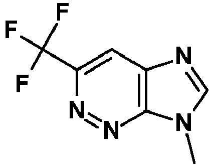 7-Methyl-3-(trifluormethyl)-7H-imidazo[4,5-c]pyridazin

Ein weiterer Gegenstand der vorliegenden Erfindung sind Verbindungen der Formel (IIIa) 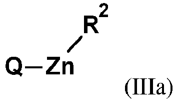 in welcher
- Q: für ein Strukturelement 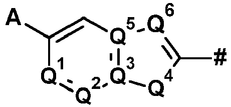 steht, wobei das Zeichen # die Bindung zum Rest des Moleküls anzeigt und
Q¹ für N oder CR⁶ steht,
Q² für N oder CR⁶ steht,
Q³ für N oder C steht,
Q⁴ für O, S, N oder NR⁷ steht,
Q⁵ für N oder C steht,
Q⁶ für N oder CH steht,
R⁶ für Wasserstoff, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht,
R⁷ für (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht,
A für Wasserstoff, Cyano, Halogen, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl oder Di-(C₁-C₄)alkylaminosulfonylsteht steht,
oder A für -O-CF₂-O- steht und gemeinsam mit Q¹ und dem Kohlenstoffatom, an das es gebunden ist, einen fünfgliedrigen Ring bildet, wobei Q¹ für Kohlenstoff steht, und
R² für Halogen oder -O-Pivaloyl steht.

Vorzugsweise stehen Q¹, Q², Q³, Q⁴, Q⁵ und Q⁶ für nicht mehr als insgesamt fünf Stickstoffatome und weiter bevorzugt für nicht mehr als insgesamt vier Stickstoffatome.

Bevorzugte, besonders bevorzugte und ganz besonders bevorzugte Bedeutungen der in den vorstehend erwähnten Verbindungen der Formel (IIIa) aufgeführten Reste werden im Folgenden erläutert.
- Q: steht bevorzugt für ein Strukturelement aus der Reihe Q1 bis Q15,
- R⁷: steht bevorzugt für (C₁-C₄)Alkyl, (C₁-C₄)Halogenalkyl, (C₁-C₄)Alkoxy-(C₁-C₄)alkyl, (C₁-C₄)Halogenalkoxy-(C₁-C₄)alkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl,
- A: steht bevorzugt für Fluor, Chlor, Brom, Fluormethyl, Difluormethyl, Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethoxy, Difluorchlormethoxy, Dichlorfluormethoxy, Trifluormethylthio, Trifluormethylsulfinyl oder Trifluormethylsulfonyl, und
- R²: steht bevorzugt für Halogen, insbesondere Chlor, Brom oder Iod.
- Q: steht besonders bevorzugt für ein Strukturelement aus der Reihe Q2, Q3, Q10, Q12, Q14 oder Q15,
- R⁷: steht besonders bevorzugt für (C₁-C₄)Alkyl oder (C₁-C₄)Alkyoxy-(C₁-C₄)alkyl,
- A: steht besonders bevorzugt für Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethylthio, Trifluormethylsulfinyl oder Trifluormethylsulfonyl, und
- R²: steht besonders bevorzugt für Chlor.
- Q: steht ganz besonders bevorzugt für das Strukturelement Q3 oder Q14,
- R⁷: steht ganz besonders bevorzugt für Methyl, Ethyl, n-Propyl oder iso-Propyl, insbesondere Methyl,
- A: steht ganz besonders bevorzugt für Trifluormethyl, und
- R²: steht ganz besonders bevorzugt für Chlor.

Die vorstehend aufgeführten Restedefinitionen können untereinander, also auch zwischen den jeweiligen Vorzugsbereichen, beliebig kombiniert werden.

Erfindungsgemäß bevorzugt werden solche Verbindungen, in welchen eine Kombination der vorstehend als bevorzugt aufgeführten Bedeutungen vorliegt.

Erfindungsgemäß besonders bevorzugt werden solche Verbindungen, in welchen eine Kombination der vorstehend als besonders bevorzugt aufgeführten Bedeutungen vorliegt.

Erfindungsgemäß ganz besonders bevorzugt werden solche Verbindungen, in welchen eine Kombination der vorstehend als ganz besonders bevorzugt aufgeführten Bedeutungen vorliegt.

Die Verbindungen der Formel (IIIa) können ferner alleine oder als Alkali- oder Erdalkalihalogenidkomplex, vorzugsweise als Lithiumchloridkomplex, vorliegen.

Beispiele für derartige ganz besonders bevorzugte Verbindungen sind:

| | | |
|---|---|---|
| IIIa-1 : | 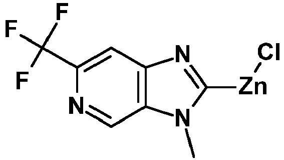 | 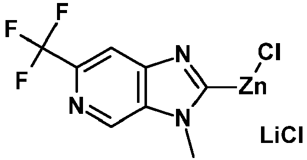 |
| | Chlor[3-methyl-6-(trifluormethyl)-3H-imidazo [4,5-c]pyridin-2-yl] zink | Chlor[3-methyl-6-(trifluormethyl)-3H-imidazo[4,5-c]pyridin-2-yl]zink |
| | | Lithiumchloridkomplex |
| IIIa-2: | 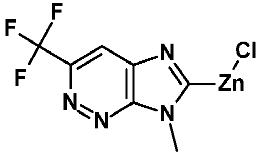 | 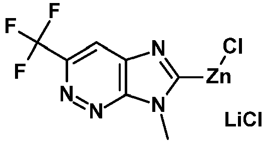 |
| | Chlor[3-methyl-6-(trifluormethyl)-3H-imidazo[4,5-c]pyridazin-2-yl]zink | Chlor[3-methyl-6-(trifluormethyl)-3H-imidazo [4,5-c]pyridazin-2-yl] zink Lithiumchloridkomplex |

Ein weiterer Gegenstand der vorliegenden Erfindung sind Verbindungen der Formel (IIIb)

Q-Zn-Q (IIIb)

in welcher
- Q: für ein Strukturelement 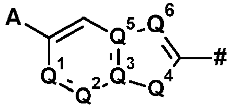 steht, wobei das Zeichen # die Bindung zum Rest des Moleküls anzeigt und
Q¹ für N oder CR⁶ steht,
Q² für N oder CR⁶ steht,
Q³ für N oder C steht,
Q⁴ für O, S, N oder NR⁷ steht,
Q⁵ für N oder C steht,
Q⁶ für N oder CH steht,
R⁶ für Wasserstoff, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht,
R⁷ für (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht, und
A für Wasserstoff, Cyano, Halogen, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl oder Di-(C₁-C₄)alkylaminosulfonylsteht steht,
oder A für -O-CF₂-O- steht und gemeinsam mit Q¹ und dem Kohlenstoffatom, an das es gebunden ist, einen fünfgliedrigen Ring bildet, wobei Q¹ für Kohlenstoff steht.

Vorzugsweise stehen Q¹, Q², Q³, Q⁴, Q⁵ und Q⁶ für nicht mehr als insgesamt fünf Stickstoffatome und weiter bevorzugt für nicht mehr als insgesamt vier Stickstoffatome.

Bevorzugte, besonders bevorzugte und ganz besonders bevorzugte Bedeutungen der in den vorstehend erwähnten Verbindungen der Formel (IIIb) aufgeführten Reste werden im Folgenden erläutert.
- Q: steht bevorzugt für ein Strukturelement aus der Reihe Q1 bis Q15,
- R⁷: steht bevorzugt für (C₁-C₄)Alkyl, (C₁-C₄)Halogenalkyl, (C₁-C₄)Alkoxy-(C₁-C₄)alkyl, (C₁-C₄)Halogenalkoxy-(C₁-C₄)alkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl, und
- A: steht bevorzugt für Fluor, Chlor, Brom, Fluormethyl, Difluormethyl, Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethoxy, Difluorchlormethoxy, Dichlorfluormethoxy, Trifluormethylthio, Trifluormethylsulfinyl oder Trifluormethylsulfonyl.
- Q: steht besonders bevorzugt für ein Strukturelement aus der Reihe Q2, Q3, Q10, Q12, Q14 oder Q15,
- R⁷: steht besonders bevorzugt für (C₁-C₄)Alkyl oder (C₁-C₄)Alkyoxy-(C₁-C₄)alkyl, und
- A: steht besonders bevorzugt für Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethylthio, Trifluormethylsulfinyl oder Trifluormethylsulfonyl.
- Q: steht ganz besonders bevorzugt für das Strukturelement Q3 oder Q14,
- R⁷: steht ganz besonders bevorzugt für Methyl, Ethyl, n-Propyl oder iso-Propyl, insbesondere Methyl, und
- A: steht ganz besonders bevorzugt für Trifluormethyl.

Die vorstehend aufgeführten Restedefinitionen können untereinander, also auch zwischen den jeweiligen Vorzugsbereichen, beliebig kombiniert werden.

Erfindungsgemäß bevorzugt werden solche Verbindungen, in welchen eine Kombination der vorstehend als bevorzugt aufgeführten Bedeutungen vorliegt.

Erfindungsgemäß besonders bevorzugt werden solche Verbindungen, in welchen eine Kombination der vorstehend als besonders bevorzugt aufgeführten Bedeutungen vorliegt.

Erfindungsgemäß ganz besonders bevorzugt werden solche Verbindungen, in welchen eine Kombination der vorstehend als ganz besonders bevorzugt aufgeführten Bedeutungen vorliegt.

Die Verbindungen der Formel (IIIb) können ferner alleine oder als Alkali- oder Erdalkalihalogenidkomplex, vorzugsweise als Lithiumchloridkomplex, vorliegen.

Ein weiterer Gegenstand der vorliegenden Erfindung sind Verbindungen der Formel (II) 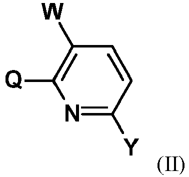 in welcher (Ausgestaltung II-1-1)
- Q: für ein Strukturelement 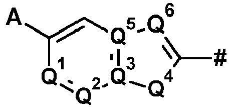 steht, wobei das Zeichen # die Bindung zum Rest des Moleküls anzeigt und
Q¹ für N oder CR⁶ steht,
Q² für N oder CR⁶ steht,
Q³ für N oder C steht,
Q⁴ für O, S, N oder NR⁷ steht,
Q⁵ für N oder C steht,
Q⁶ für N oder CH steht,
R⁶ für Wasserstoff, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht,
R⁷ für (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht,
A für Wasserstoff, Cyano, Halogen, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl oder Di-(C₁-C₄)alkylaminosulfonylsteht steht,
oder A für -O-CF₂-O- steht und gemeinsam mit Q¹ und dem Kohlenstoffatom, an das es gebunden ist, einen fünfgliedrigen Ring bildet, wobei Q¹ für Kohlenstoff steht,
- W: für Halogen steht, und
- Y: für Halogen, CO₂R¹ oder NO₂ steht, wobei R¹ für (C₁-C₆)-Alkyl oder (C₁-C₆)-Haloalkyl steht.

Eine alternative Ausführungsform (Ausgestaltung II-1-2) sind Verbindungen der Formel (II), in welcher
- Q: für ein Strukturelement 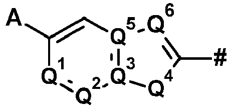 steht, wobei das Zeichen # die Bindung zum Rest des Moleküls anzeigt und
Q¹ für N oder CR⁶ steht,
Q² für N oder CR⁶ steht,
Q³ für N oder C steht,
Q⁴ für O, S, N oder NR⁷ steht,
Q⁵ für N oder C steht,
Q⁶ für N oder CH steht,
R⁶ für Wasserstoff, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht,
R⁷ für (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht,
A für Wasserstoff, Cyano, Halogen, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl oder Di-(C₁-C₄)alkylaminosulfonylsteht steht,
oder A für -O-CF₂-O- steht und gemeinsam mit Q¹ und dem Kohlenstoffatom, an das es gebunden ist, einen fünfgliedrigen Ring bildet, wobei Q¹ für Kohlenstoff steht,
- W: für Fluor oder Brom steht, und
- Y: für Halogen, CO₂R¹ oder NO₂ steht, wobei R¹ für (C₁-C₆)-Alkyl oder (C₁-C₆)-Haloalkyl steht.

Vorzugsweise stehen Q¹, Q², Q³, Q⁴, Q⁵ und Q⁶ in Ausgestaltung II-1-1 und Ausgestaltung II-1 -2 für nicht mehr als insgesamt fünf Stickstoffatome und weiter bevorzugt für nicht mehr als insgesamt vier Stickstoffatome.

Bevorzugte (Ausgestaltungen II-2-1 und II-2-2), besonders bevorzugte (Ausgestaltungen II-3-1 und II-3-2) und ganz besonders bevorzugte (Ausgestaltung II-4-1) Bedeutungen der in der vorstehend erwähnten Formel (II) aufgeführten Reste werden im Folgenden erläutert.
Ausgestaltung II-2-1:
   - Q: steht bevorzugt für ein Strukturelement aus der Reihe Q1 bis Q15,
   - R⁷: steht bevorzugt für (C₁-C₄)Alkyl, (C₁-C₄)Halogenalkyl, (C₁-C₄)Alkoxy-(C₁-C₄)alkyl, (C₁-C₄)Halogenalkoxy-(C₁-C₄)alkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl,
   - A: steht bevorzugt für Fluor, Chlor, Brom, Fluormethyl, Difluormethyl, Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethoxy, Difluorchlormethoxy, Dichlorfluormethoxy, Trifluormethylthio, Trifluormethylsulfinyl oder Trifluormethylsulfonyl,
   - W: steht bevorzugt für Fluor, Chlor oder Brom, und
   - Y: steht bevorzugt für Fluor, Chlor, Brom, CO₂R¹ oder NO₂, wobei R¹ für (C₁-C₄)-Alkyl.
Ausgestaltung II-2-2:
   - Q: steht bevorzugt für ein Strukturelement aus der Reihe Q1 bis Q15,
   - R⁷: steht bevorzugt für (C₁-C₄)Alkyl, (C₁-C₄)Halogenalkyl, (C₁-C₄)Alkoxy-(C₁-C₄)alkyl, (C₁-C₄)Halogenalkoxy-(C₁-C₄)alkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl,
   - A: steht bevorzugt für Fluor, Chlor, Brom, Fluormethyl, Difluormethyl, Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethoxy, Difluorchlormethoxy, Dichlorfluormethoxy, Trifluormethylthio, Trifluormethylsulfinyl oder Trifluormethylsulfonyl,
   - W: steht bevorzugt für Fluor oder Brom, und
   - Y: steht bevorzugt für Fluor, Chlor, Brom, CO₂R¹ oder NO₂, wobei R¹ für (C₁-C₄)-Alkyl steht.
Ausgestaltung II-3-1:
   - Q: steht besonders bevorzugt für ein Strukturelement aus der Reihe Q2, Q3, Q10, Q12, Q14 oder Q15,
   - R⁷: steht besonders bevorzugt für (C₁-C₄)Alkyl oder (C₁-C₄)Alkyoxy-(C₁-C₄)alkyl,
   - A: steht besonders bevorzugt für Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethylthio, Trifluormethylsulfinyl oder Trifluormethylsulfonyl,
   - W: steht besonders bevorzugt für Fluor oder Chlor, insbesondere Fluor, und
   - Y: steht besonders bevorzugt für Chlor, Brom, CO₂R¹ oder NO₂, wobei R¹ für (C₁-C₄)-Alkyl steht.
Ausgestaltung II-3-2:
   - Q: steht besonders bevorzugt für ein Strukturelement aus der Reihe Q2, Q3, Q10, Q12, Q14 oder Q15,
   - R⁷: steht besonders bevorzugt für (C₁-C₄)Alkyl oder (C₁-C₄)Alkyoxy-(C₁-C₄)alkyl,
   - A: steht besonders bevorzugt für Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethylthio, Trifluormethylsulfinyl oder Trifluormethylsulfonyl,
   - W: steht besonders bevorzugt für Fluor, und
   - Y: steht besonders bevorzugt für Chlor, Brom, CO₂R¹ oder NO₂, wobei R¹ für (C₁-C₄)-Alkyl steht.
Ausgestaltung II-4-1:
   - Q: steht ganz besonders bevorzugt für das Strukturelement Q3 oder Q14,
   - R⁷: steht ganz besonders bevorzugt für Methyl, Ethyl, n-Propyl oder iso-Propyl, insbesondere Methyl,
   - A: steht ganz besonders bevorzugt für Trifluormethyl,
   - W: steht ganz besonders bevorzugt für Fluor, und
   - Y: steht ganz besonders bevorzugt für Chlor, Brom, CO₂R¹ oder NO₂, wobei R¹ für Methyl steht.

Die vorstehend aufgeführten Restedefinitionen können untereinander, also auch zwischen den jeweiligen Vorzugsbereichen, beliebig kombiniert werden.

Erfindungsgemäß bevorzugt werden solche Verbindungen, in welchen eine Kombination der vorstehend als bevorzugt aufgeführten Bedeutungen vorliegt.

Erfindungsgemäß besonders bevorzugt werden solche Verbindungen, in welchen eine Kombination der vorstehend als besonders bevorzugt aufgeführten Bedeutungen vorliegt.

Erfindungsgemäß ganz besonders bevorzugt werden solche Verbindungen, in welchen eine Kombination der vorstehend als ganz besonders bevorzugt aufgeführten Bedeutungen vorliegt.

Beispiele für derartige ganz besonders bevorzugte Verbindungen sind: 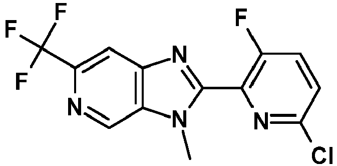 2-(6-Chlor-3-fluorpyridin-2-yl)-3-methyl-6-(trifluormethyl)-3H-imidazo[4,5-c]pyridin 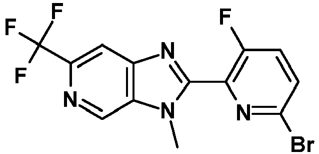 2-(6-Brom-3 -fluorpyridin-2-yl)-3-methyl-6-(trifluormethyl)-3H-imidazo[4,5-c]pyridin 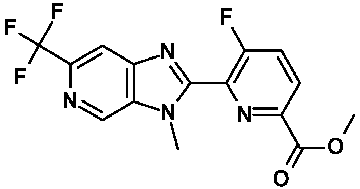 Methyl-5-fluor-6-[3-methyl-6-(trifluormethyl)-3H-imidazo[4,5-c]pyridin-2-yllpyridin-2-carboxylat 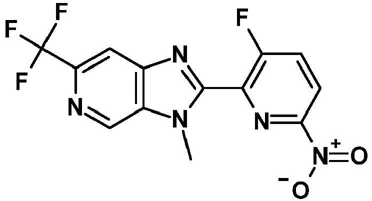 2-(3-Fluor-6-nitropyridin-2-yl)-3-methyl-6-(trifluormethyl)-3H-imidazo[4,5-c]pyridin 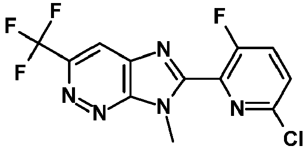 6-(6-Chlor-3-fluorpyridin-2-yl)-7-methyl-3-(trifluormethyl)-7H-imidazo[4,5-c]pyridazin 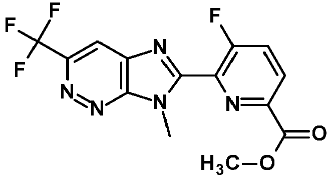 Methyl-5-fluor-6-[7-methyl-3-(trifluormethyl)-7H-imidazo[4,5-c]pyridazin-6-yl]pyridin-2-carboxylat 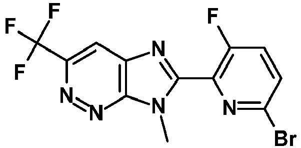 6-(6-Brom-3-fluorpyridin-2-yl)-7-methyl-3-(trifluormethyl)-7H-imidazo[4,5-c]pyridazin

Die vorliegende Erfindung wird anhand der nachfolgenden Beispiele näher erläutert, wobei die Beispiele nicht in einer die Erfindung einschränkenden Weise zu interpretieren sind.

### Beispiel 1

### Synthese von 3-Methyl-6-(trifluormethyl)-3H-imidazo[4,5-c]pyridin

N3-Methyl-6-(trifluormethyl)pyridin-3,4-diamin (500 mg, 2,6 mmol), gelöst in Ameisensäure (4 ml, 106 mmol), wurde mit Mikrowellen für 1 Stunde bei 150°C erhitzt. Nach üblicher Aufarbeitung durch Zugabe von gesättigter Ammoniumchlorid-Lösung wurde das Reaktionsgemisch mit Ethylacetat extrahiert, die vereinigten organischen Phasen über Na₂SO₄ getrocknet und im Membranpumpenvakuum eingeengt. Nach säulenchromatographischer Reinigung (Ethylacetat/Cyclohexan) erhielt man 3-Methyl-6-(trifluormethyl)-3H-imidazo[4,5-c]pyridin (480 mg, 91%) als weißen Feststoff. HPLC-MS: logP = 1,09; Masse (m/z+1): 202,0; 1HNMR (D6-DMSO): δ 9,14 (s, 1H), 8,61 (s, 1H), 8,19 (s, 1H), 4,02 (s, 3H).

### Beispiel 2

### Synthese von 2-(6-Chlor-3-fluorpyridin-2-yl)-3-methyl-6-(trifluormethyl)-3H-imidazo[4,5-c]pyridin:

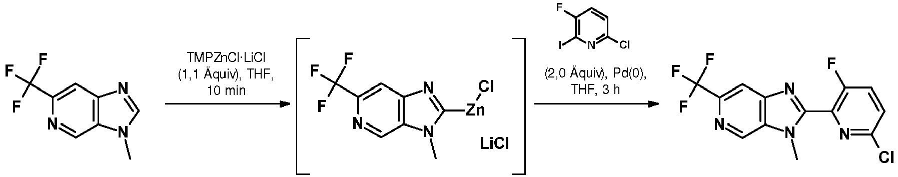

3-Methyl-6-(trifluormethyl)-3H-imidazo[4,5-c]pyridin (201 mg, 1,0 mmol), gelöst in THF (2 ml), wurde mit TMPZnCl·LiCl (1,31 M in THF, 0,84 ml, 1.1 mmol) bei 25°C unter Argon versetzt; diese Reaktionslösung wurde 10 min gerührt. Anschließend wurde 6-Chlor-3-fluor-2-iodpyridin (515 mg, 2 mmol in 4 ml THF) und Tetrakis(triphenylphosphin)palladium(0) (115 mg, 0,1 mmol) bei 25°C zugegeben und die Lösung während 3 Stunden bei 65°C weitergerührt. Nach üblicher Aufarbeitung durch Zugabe von gesättigter Ammoniumchlorid-Lösung wurde das Reaktionsgemisch mit Ethylacetat extrahiert, die vereinigten organischen Phasen über Na₂SO₄ getrocknet und im Membranpumpenvakuum eingeengt. Nach säulenchromatographischer Reinigung (Ethylacetat/Cyclohexan) erhielt man 2-(6-Chlor-3-fluorpyridin-2-yl)-3-methyl-6-(trifluormethyl)-3H-imidazo[4,5-c]pyridin (177 mg, 52%) als weißen Feststoff. HPLC-MS: logP = 2,38; Masse (m/z): 331,0; 1HNMR (D6-DMSO): δ 9,29 (s, 1H), 8,32 (s, 1H), 8,19 (t, 1H), 7,91 (dd, 1H), 4,14 (s, 3H).

### Beispiel 3

### Synthese von Methyl-5-fluor-6-[3-methyl-6-(trifluormethyl)-3H-imidazo[4,5-c]pyridin-2-yl]pyridin-2-carboxylat

3-Methyl-6-(trifluormethyl)-3H-imidazo[4,5-c]pyridin (201 mg, 1,0 mmol), gelöst in THF (2 ml), wurde mit TMPZnCl·LiCl (1,31 M in THF, 0,84 ml, 1.1 mmol) bei 25°C unter Argon versetzt; diese Reaktionslösung wurde 10 min gerührt. Anschließend wurde Methyl-5-fluor-6-iodpyridin-2-carboxylat (562 mg, 2 mmol in 4 ml THF) und Tetrakis(triphenylphosphin)palladium(0) (115 mg, 0,1 mmol) bei 25°C zugegeben und die Lösung während 3 Stunden bei 65°C weitergerührt. Nach üblicher Aufarbeitung durch Zugabe von gesättigter Ammoniumchlorid-Lösung wurde das Reaktionsgemisch mit Ethylacetat extrahiert, die vereinigten organischen Phasen über Na₂SO₄ getrocknet und im Membranpumpenvakuum eingeengt. Nach säulenchromatographischer Reinigung (Ethylacetat/Cyclohexan) erhielt man Methyl-5-fluor-6-[3-methyl-6-(trifluormethyl)-3H-imidazo[4,5-c]pyridin-2-yl]pyridin-2-carboxylat (160 mg, 45%) als weißen Feststoff. HPLC-MS: logP = = 2,03; Masse (m/z+1): 355,1; 1HNMR (D6-DMSO): 9,30 (s, 1H), 8,38 (dd, 1H), 8,34 (s, 1H), 8,24 (dd, 1H), 4,19 (s, 3H), 3,93 (s, 3H).

### Beispiel 4

### Synthese von 2-(6-Brom-3-fluorpyridin-2-yl)-3-methyl-6-(trifluormethyl)-3H-imidazo[4,5-c]pyridin:

3-Methyl-6-(trifluormethyl)-3H-imidazo[4,5-c]pyridin (100 mg, 0,5 mmol), gelöst in THF (2 ml), wurde mit TMPZnCl·LiCl (1,31 M in THF, 0.52 ml, 0,55 mmol) bei 25°C unter Argon versetzt; diese Reaktionslösung wurde 10 min gerührt. Anschließend wurde 6-Brom-3-fluor-2-iodpyridin (301 mg, 1 mmol in 2 ml THF) und Tetrakis(triphenylphosphin)palladium(0) (58 mg, 0,05 mmol) bei 25°C zugegeben und die Lösung während 3 Stunden bei 65°C weitergerührt. Nach üblicher Aufarbeitung durch Zugabe von gesättigter Ammoniumchlorid-Lösung wurde das Reaktionsgemisch mit Ethylacetat extrahiert, die vereinigten organischen Phasen über Na₂SO₄ getrocknet und im Membranpumpenvakuum eingeengt. Nach säulenchromatographischer Reinigung (Ethylacetat/Cyclohexan) erhielt man 2-(6-Brom-3-fluorpyridin-2-yl)-3-methyl-6-(trifluormethyl)-3H-imidazo[4,5-c]pyridin (101 mg, 54%) als weißen Feststoff. HPLC-MS: logP = 2,51; Masse (m/z+1): 376,9; 1HNMR (D6-DMSO): δ 9,29 (s, 1H), 8,33 (s, 1H), 8,05 (m, 2H), 4,13 (s, 3H).

### Beispiel 5

### Synthese von 7-Methyl-3-(trifluormethyl)-7H-imidazo[4,5-c]pyridazin

N3-Methyl-6-(trifluormethyl)pyridazin-3,4-diamin (192 mg, 1,0 mmol), gelöst in Ameisensäure (0,4 ml, 106 mmol), wurde mit Mikrowellen für 2 Stunden bei 150°C erhitzt. Nach üblicher Aufarbeitung durch Zugabe von gesättigter Ammoniumchlorid-Lösung wurde das Reaktionsgemisch mit Ethylacetat extrahiert, die vereinigten organischen Phasen über Na₂SO₄ getrocknet und im Membranpumpenvakuum eingeengt. Nach säulenchromatographischer Reinigung (Ethylacetat/Cyclohexan) erhielt man 7-Methyl-3-(trifluormethyl)-7H-imidazo[4,5-c]pyridazin (149 mg, 74%) als weißen Feststoff. HPLC-MS: logP = 0,95; Masse (m/z+1): 203,1; 1HNMR (D6-DMSO): δ 8,97 (s, 1H), 8,62 (s, 1H), 4,08 (s, 3H).

### Beispiel 6

### Synthese von 6-(6-Chlor-3-fluorpyridin-2-yl)-7-methyl-3-(trifluormethyl)-7H-imidazo[4,5-c]pyridazin:

7-Methyl-3-(trifluormethyl)-7H-imidazo[4,5-c]pyridazin (202 mg, 1,0 mmol), gelöst in THF (2 ml), wurde mit TMPZnCl·LiCl (1,31 M in THF, 0,84 ml, 1,1 mmol) bei 25°C unter Argon versetzt; diese Reaktionslösung wurde 10 min gerührt. Anschließend wurde 6-Chlor-3-fluor-2-iodpyridin (515 mg, 2 mmol in 4 ml THF) und Tetrakis(triphenylphosphin)palladium(0) (115 mg, 0,1 mmol) bei 25°C zugegeben und die Lösung während 3 Stunden bei 65°C weitergerührt. Nach üblicher Aufarbeitung durch Zugabe von gesättigter Ammoniumchlorid-Lösung wurde das Reaktionsgemisch mit Ethylacetat extrahiert, die vereinigten organischen Phasen über Na₂SO₄ getrocknet und im Membranpumpenvakuum eingeengt. Nach säulenchromatographischer Reinigung (Ethylacetat/Cyclohexan) erhielt man 6-(6-Chlor-3-fluorpyridin-2-yl)-7-methyl-3-(trifluormethyl)-7H-imidazo[4,5-c]pyridazin (125 mg, 38%) als weißen Feststoff. HPLC-MS: logP = 2,46; Masse (m/z): 332,0; 1HNMR (D6-DMSO): δ 8,77 (s, 1H), 8,23 (t, 1H), 7,98 (dd, 1H), 4,25 (s, 3H).

### Beispiel 7

### Synthese von Methyl-5-fluor-6-[7-methyl-3-(trifluormethyl)-7H-imidazo[4,5-c]pyridazin-6-yl]pyridin-2-carboxylat:

7-Methyl-3-(trifluormethyl)-7H-imidazo[4,5-c]pyridazin (202 mg, 1,0 mmol), gelöst in THF (2 ml), wurde mit TMPZnCl·LiCl (1,31 M in THF, 0,84 ml, 1,1 mmol) bei 25°C unter Argon versetzt; diese Reaktionslösung wurde 10 min gerührt. Anschließend wurde Methyl-5-fluor-6-iodpyridin-2-carboxylat (562 mg, 2 mmol in 4 ml THF) und Tetrakis(triphenylphosphin)palladium(0) (115 mg, 0,1 mmol) bei 25°C zugegeben und die Lösung während 3 Stunden bei 65°C weitergerührt. Nach üblicher Aufarbeitung durch Zugabe von gesättigter Ammoniumchlorid-Lösung wurde das Reaktionsgemisch mit Ethylacetat extrahiert, die vereinigten organischen Phasen über Na₂SO₄ getrocknet und im Membranpumpenvakuum eingeengt. Nach säulenchromatographischer Reinigung (Ethylacetat/Cyclohexan) erhielt man Methyl-5-fluor-6-[7-methyl-3-(trifluormethyl)-7H-imidazo[4,5-c]pyridazin-6-yl]pyridin-2-carboxylat (208 mg, 59%) als weißen Feststoff. HPLC-MS: logP = = 2,09; Masse (m/z+1): 356,0; 1HNMR (D6-DMSO): 8,78 (s, 1H), 8,43 (dd, 1H), 8,28 (t, 1H), 4,32 (s, 3H), 3,96 (s, 3H).

### Beispiel 8

### Synthese von 6-(6-Brom-3-fluorpyridin-2-yl)-7-methyl-3-(trifluormethyl)-7H-imidazo[4,5-c]pyridazin:

7-Methyl-3-(trifluormethyl)-7H-imidazo[4,5-c]pyridazin (340 mg, 1,7 mmol), gelöst in THF (2 ml), wurde mit TMPZnCl·LiCl (1,31 M in THF, 1,41 ml, 1,85 mmol) bei 25°C unter Argon versetzt; diese Reaktionslösung wurde 10 min gerührt. Anschließend wurde 6-Brom-3-fluor-2-iodpyridin (1,016 g, 3,36 mmol in 4 ml THF) und Tetrakis(triphenylphosphin)palladium(0) (195 mg, 0,16 mmol) bei 25°C zugegeben und die Lösung während 3 Stunden bei 65°C weitergerührt. Nach üblicher Aufarbeitung durch Zugabe von gesättigter Ammoniumchlorid-Lösung wurde das Reaktionsgemisch mit Ethylacetat extrahiert, die vereinigten organischen Phasen über Na₂SO₄ getrocknet und im Membranpumpenvakuum eingeengt. Nach säulenchromatographischer Reinigung (Ethylacetat/Cyclohexan) erhielt man 6-(6-Brom-3-fluorpyridin-2-yl)-7-methyl-3-(trifluormethyl)-7H-imidazo[4,5-c]pyridazin (408 mg, 62%) als weißen Feststoff. HPLC-MS: logP = 2,58; Masse (m/z+1): 375,9; 1HNMR (D6-DMSO): δ 8,77 (s, 1H), 8,10 (m, 2H), 4,25 (s, 3H)

Die vorliegende Erfindung betrifft ein Verfahren zur Herstellung von halogenierten Pyridinderivaten der Formel (11) ausgehend von Verbindungen der Struktur Q-H über Zwischenstufen der Formel (IIIa) oder (IIIb)

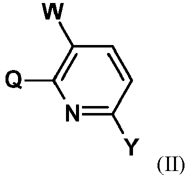

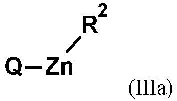

**Q-Zn-Q** (IIIb),

in welchen
Q für ein Strukturelement 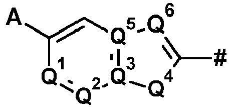 steht, wobei das Zeichen # die Bindung zum Rest des Moleküls anzeigt und A, Q¹, Q², Q³, Q⁴, Q⁵ und Q⁶ die in der Beschreibung angegebenen Bedeutungen haben,
W für Halogen steht,
Y für Halogen oder CO₂R¹ steht, wobei R¹ für (C₁-C₆)-Alkyl oder (C₁-C₆)-Haloalkyl steht, und
R² für Halogen steht.

## Patentansprüche

1. Verfahren zur Herstellung von Verbindungen der Formel (II) 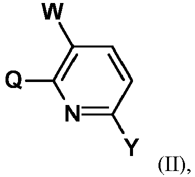 in welcher
Q für ein Strukturelement 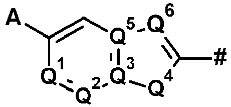
steht, wobei das Zeichen # die Bindung zum Rest des Moleküls anzeigt und
Q¹ für N oder CR⁶ steht,
Q² für N oder CR⁶ steht,
Q³ für N oder C steht,
Q⁴ für O, S, N oder NR⁷ steht,
Q⁵ für N oder C steht,
Q⁶ für N oder CH steht,
R⁶ für Wasserstoff, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht,
R⁷ für (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht, und
A für Wasserstoff, Cyano, Halogen, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl oder Di-(C₁-C₄)alkylaminosulfonyl steht,
oder A für -O-CF₂-O- steht und gemeinsam mit Q¹ und dem Kohlenstoffatom, an das es gebunden ist, einen fünfgliedrigen Ring bildet, wobei Q¹ für Kohlenstoff steht,
W für Halogen steht, und
Y für Halogen, CO₂R¹ oder NO₂ steht, wobei R¹ für (C₁-C₆)-Alkyl oder (C₁-C₆)-Haloalkyl steht,
**dadurch gekennzeichnet, dass** in einem ersten Verfahrensschritt a) eine Verbindung Q-H, in welcher Q die oben genannte Bedeutung hat,
mit einer Zink-metallorganischen Base der Struktur (NR³R⁴)-Zn-R² oder (NR³R⁴)₂-Zn, in welcher
R² für Halogen oder -O-Pivaloyl steht und
R³ und R⁴ gemeinsam eine -(CH₂)₄-, -(CH₂)₅- oder -(CH₂)₂O(CH₂)₂-Gruppe bilden, wobei jede dieser Gruppen optional durch 1, 2, 3 oder 4 R⁵-Radikale substituiert sein kann und R⁵ ausgewählt ist aus der Gruppe bestehend aus Methyl, Ethyl, n-Propyl und i-Propyl,
zu einer Verbindung der Formel (IIIa) oder der Formel (IIIb) umgesetzt wird, 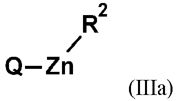
Q-Zn-Q (IIIb),
in welcher Q und R² jeweils die oben genannten Bedeutungen haben,
und diese Verbindung der Formel (IIIa) oder (IIIb) in einem zweiten Verfahrensschritt b) mit einer Verbindung der Formel (I) 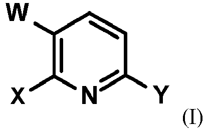 in welcher X für Halogen steht und W und Y jeweils die oben genannten Bedeutungen haben, in Gegenwart eines Katalysators zur Verbindung der Formel (II) umgesetzt wird.

2. Verfahren gemäß Anspruch 1, **dadurch gekennzeichnet, dass**
Q für ein Strukturelement aus der Reihe Q1 bis Q15 steht, 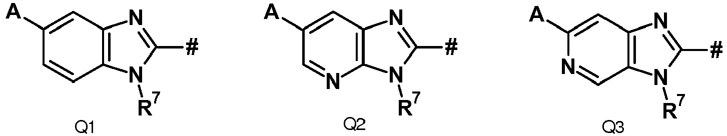 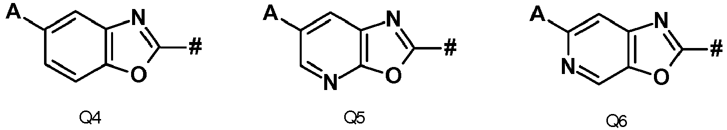 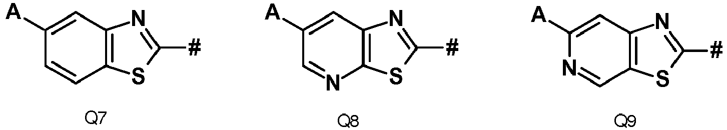 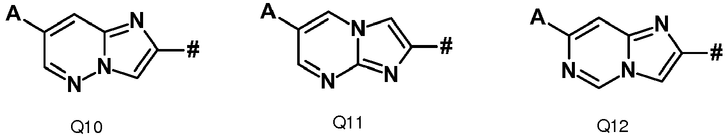 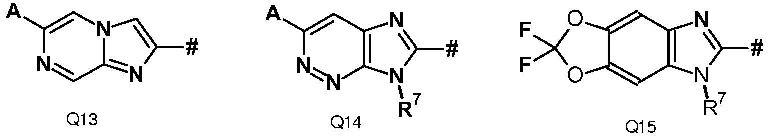
R⁷ für (C₁-C₄)Alkyl, (C₁-C₄)Halogenalkyl, (C₁-C₄)Alkoxy-(C₁-C₄)alkyl, (C₁-C₄)Halogenalkoxy-(C₁-C₄)alkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl,
A für Fluor, Chlor, Brom, Fluormethyl, Difluormethyl, Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethoxy, Difluorchlormethoxy, Dichlorfluormethoxy, Trifluormethylthio, Trifluormethylsulfinyl oder Trifluormethylsulfonyl steht,
W für Fluor, Chlor oder Brom steht,
R² für Halogen, insbesondere Chlor, Brom oder Iod, steht,
X für Halogen, insbesondere Brom oder Iod, steht, und
Y für Fluor, Chlor, Brom, CO₂R¹ oder NO₂ steht, wobei R¹ für (C₁-C₄)-Alkyl steht.

3. Verfahren gemäß einem der Ansprüche 1 oder 2, **dadurch gekennzeichnet, dass**
Q für ein Strukturelement aus der Reihe Q2, Q3, Q10, Q12, Q14 oder Q15 steht,
R⁷ für (C₁-C₄)Alkyl oder (C₁-C₄)Alkyoxy-(C₁-C₄)alkyl steht,
A für Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethylthio, Trifluormethylsulfinyl oder Trifluormethylsulfonyl steht,
W für Fluor oder Chlor, insbesondere Fluor, steht,
R² für Chlor steht,
X für Brom oder Iod, insbesondere Iod, steht, und
Y für Chlor, Brom, CO₂R¹ oder NO₂ steht, wobei R¹ für (C₁-C₄)-Alkyl steht.

4. Verfahren gemäß einem der Ansprüche 1 bis 3, **dadurch gekennzeichnet, dass**
Q für das Strukturelement Q3 oder Q14 steht,
R⁷ für Methyl, Ethyl, n-Propyl oder iso-Propyl, insbesondere Methyl, steht,
A für Trifluormethyl steht,
W für Fluor steht,
R² für Chlor steht,
X für Iod steht, und
Y für Chlor, Brom, CO₂R¹ oder NO₂ steht, wobei R¹ für Methyl steht.

5. Verfahren gemäß einem der Ansprüche 1 bis 4, **dadurch gekennzeichnet, dass**
R³ und R⁴ gemeinsam eine -(CH₂)₅-Gruppe bilden, die durch 4 Methylgruppen substituiert ist.

6. Verfahren gemäß einem der Ansprüche 1 bis 5, **dadurch gekennzeichnet, dass** es sich bei der Zink-metallorganischen Base um eine Verbindung der Formel (V) handelt
(V) (TMP)ₓZnCl₂₋ₓ,
worin x für die Zahl 1 oder 2 steht.

7. Verfahren gemäß einem der Ansprüche 1 bis 6, **dadurch gekennzeichnet, dass** die Zinkmetallorganische Base in Verbindung mit einem Alkali- oder Erdalkalihalogenid, vorzugsweise Lithiumchlorid oder Magnesiumchlorid, vorliegt.

8. Verfahren gemäß einem der Ansprüche 1 bis 7, **dadurch gekennzeichnet, dass** die Zinkmetallorganische Base in einer Gesamtmenge von 0,5 bis 5 Äquivalenten, bezogen auf die Verbindung Q-H, eingesetzt wird.

9. Verfahren gemäß einem der Ansprüche 1 bis 8, **dadurch gekennzeichnet, dass** die Verbindung der Formel (I) in einer Gesamtmenge von 0,5 bis 10,0 Äquivalenten, bezogen auf die Verbindung Q-H, eingesetzt wird.

10. Verfahren gemäß einem der Ansprüche 1 bis 9, **dadurch gekennzeichnet, dass** es sich bei dem Katalysator um eine Palladiumverbindung handelt.

11. Verfahren gemäß einem der Ansprüche 1 bis 10, **dadurch gekennzeichnet, dass** es sich bei dem Katalysator um Tetrakis(triphenylphosphine)palladium(0) handelt.

12. Verfahren gemäß einem der Ansprüche 1 bis 11, **dadurch gekennzeichnet, dass** es in Gegenwart eines Lösungsmittels durchgeführt wird, welches ausgewählt ist aus der Gruppe bestehend aus Tetrahydrofuran (THF), 1,4-Dioxan, Diethylether, Diglyme, Methyltertbutylether (MTBE), tert-Amyl-methylether (TAME), 2-Methyl-THF, Toluol, Xylole, Mesitylen, Ethylencarbonat, Propylencarbonat, N,N-Dimethylacetamid, N,N-Dimethylformamid (DMF), N-Methylpyrrolidon (NMP), N-Ethyl-2-pyrrolidon (NEP), N-Butyl-2-pyrrolidon (NBP); N,N'-Dimethylpropylenharnstoff (DMPU), Halogenkohlenwasserstoff, aromatischer Kohlenwasserstoff, Chlorkohlenwasserstoff, Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, 1,2-Dichlorbenzol, Chlortoluol, Trichlorbenzol; 4-Methoxybenzol, fluoriertes Aliphat, fluorierter Aromat, Trichlortrifluorethan, Benzotrifluorid und 4-Chlorbenzotrifluorid, oder einer Mischung aus mindestens zwei dieser Lösungsmittel untereinander.

13. Verfahren gemäß Anspruch 12, **dadurch gekennzeichnet, dass** das Lösungsmittel THF oder N,N-Dimethylformamid (DMF) ist.

14. Verfahren gemäß einem der Ansprüche 1 bis 13, **dadurch gekennzeichnet, dass** Verfahrensschritt a) bei einer Temperatur zwischen 0°C und 80°C durchgeführt wird.

15. Verfahren gemäß einem der Ansprüche 1 bis 13, **dadurch gekennzeichnet, dass** Verfahrensschritt b) bei einer Temperatur zwischen 40°C und 90°C durchgeführt wird.

16. Verbindung der Struktur 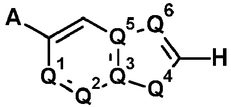 wobei
Q¹ für N oder CR⁶ steht,
Q² für N oder CR⁶ steht,
Q³ für N oder C steht,
Q⁴ für O, S, N oder NR⁷ steht,
Q⁵ für N oder C steht,
Q⁶ für N oder CH steht,
R⁶ für Wasserstoff, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht,
R⁷ für (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht, und
A für Cyano, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl oder Di-(C₁-C₄)alkylaminosulfonylsteht steht,
oder A für -O-CF₂-O- steht und gemeinsam mit Q¹ und dem Kohlenstoffatom, an das es gebunden ist, einen fünfgliedrigen Ring bildet, wobei Q¹ für Kohlenstoff steht.

17. Verbindung der Formel (IIIa) 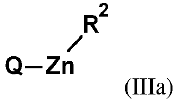 in welcher
Q für ein Strukturelement aus der Reihe Q1 bis Q15 steht,
R⁷ für (C₁-C₄)Alkyl, (C₁-C₄)Halogenalkyl, (C₁-C₄)Alkoxy-(C₁-C₄)alkyl, (C₁-C₄)Halogenalkoxy-(C₁-C₄)alkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht,
A für Fluor, Chlor, Brom, Fluormethyl, Difluormethyl, Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethoxy, Difluorchlormethoxy, Dichlorfluormethoxy, Trifluormethylthio, Trifluormethylsulfinyl oder Trifluormethylsulfonyl steht, und
R ² für Halogen, insbesondere Chlor, Brom oder Iod, steht.

18. Verbindung der Formel (IIIb)
**Q-Zn-Q** (IIIb)
in welcher
Q für ein Strukturelement aus der Reihe Q1 bis Q15 steht,
R⁷ für (C₁-C₄)Alkyl, (C₁-C₄)Halogenalkyl, (C₁-C₄)Alkoxy-(C₁-C₄)alkyl, (C₁-C₄)Halogenalkoxy-(C₁-C₄)alkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht, und
A für Fluor, Chlor, Brom, Fluormethyl, Difluormethyl, Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethoxy, Difluorchlormethoxy, Dichlorfluormethoxy, Trifluormethylthio, Trifluormethylsulfinyl oder Trifluormethylsulfonyl steht.

19. Verbindung der Formel (II) 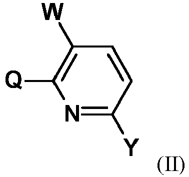 in welcher
Q für ein Strukturelement aus der Reihe Q1 bis Q15 steht,
R⁷ für (C₁-C₄)Alkyl, (C₁-C₄)Halogenalkyl, (C₁-C₄)Alkoxy-(C₁-C₄)alkyl, (C₁-C₄)Halogenalkoxy-(C₁-C₄)alkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht,
A für Fluor, Chlor, Brom, Fluormethyl, Difluormethyl, Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethoxy, Difluorchlormethoxy, Dichlorfluormethoxy, Trifluormethylthio, Trifluormethylsulfinyl oder Trifluormethylsulfonyl steht,
W für Fluor oder Brom steht, und
Y für Fluor, Chlor, Brom, CO₂R¹ oder NO₂ steht, wobei R¹ für (C₁-C₄)-Alkyl steht.

## Claims

1. Process for preparing compounds of the formula (II) 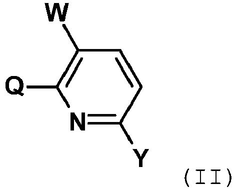 in which
Q is a structural element 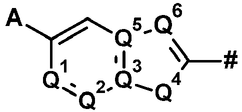
where the symbol # indicates the bond to the rest of the molecule and
Q¹ is N or CR⁶,
Q² is N or CR⁶,
Q³ is N or C,
Q⁴ is O, S, N or NR⁷,
Q⁵ is N or C,
Q⁶ is N or CH,
R⁶ is hydrogen, (C₁-C₄)alkyl, (C₁-C₄)haloalkyl, (C₁-C₄)cyanoalkyl, (C₁-C₄)hydroxyalkyl, (C₁-C₄)alkoxy-(C₁-C₄)alkyl, (C₁-C₄)haloalkoxy-(C₁-C₄)alkyl, (C₂-C₄)alkenyl, (C₂-C₄)alkenyloxy-(C₁-C₄)alkyl, (C₂-C₄)haloalkenyloxy-(C₁-C₄)alkyl, (C₂-C₄)haloalkenyl, (C₂-C₄) cyanoalkenyl, (C₂-C₄)alkynyl, (C₂-C₄) alkynyloxy-(C₁-C₄)alkyl, (C₂-C₄)haloalkynyl, (C₃-C₆)cycloalkyl, (C₃-C₆)cycloalkyl-(C₃-C₆)cycloalkyl, (C₁-C₄)alkyl-(C₃-C₆)cycloalkyl, halo(C₃-C₆)cycloalkyl, (C₁-C₄)alkylthio-(C₁-C₄)alkyl, (C₁-C₄)alkylsulphinyl-(C₁-C₄)alkyl, (C₁-C₄)alkylsulphonyl-(C₁-C₄)alkyl or (C₁-C₄)alkylcarbonyl-(C₁-C₄)alkyl,
R⁷ is (C₁-C₄)alkyl, (C₁-C₄)haloalkyl, (C₁-C₄)cyanoalkyl, (C₁-C₄)hydroxyalkyl, (C₁-C₄)alkoxy-(C₁-C₄)alkyl, (C₁-C₄)haloalkoxy-(C₁-C₄)alkyl, (C₂-C₄)alkenyl, (C₂-C₄)alkenyloxy-(C₁-C₄)alkyl, (C₂-C₄)haloalkenyloxy-(C₁-C₄)alkyl, (C₂-C₄)haloalkenyl, (C₂-C₄)cyanoalkenyl, (C₂-C₄)alkynyl, (C₂-C₄)alkynyloxy-(C₁-C₄)alkyl, (C₂-C₄)haloalkynyl, (C₃-C₆)cycloalkyl, (C₃-C₆)cycloalkyl-(C₃-C₆)cycloalkyl, (C₁-C₄)alkyl-(C₃-C₆)cycloalkyl, halo(C₃-C₆)cycloalkyl, (C₁-C₄)alkylthio-(C₁-C₄)alkyl, (C₁-C₄)alkylsulphinyl-(C₁-C₄)alkyl, (C₁-C₄)alkylsulphonyl-(C₁-C₄)alkyl or (C₁-C₄)alkylcarbonyl-(C₁-C₄)alkyl, and
A is hydrogen, cyano, halogen, (C₁-C₄)alkyl, (C₁-C₄)haloalkyl, (C₂-C₄)alkenyl, (C₂-C₄)haloalkenyl, (C₂-C₄)alkynyl, (C₂-C₄)haloalkynyl, (C₃-C₆)cycloalkyl, (C₃-C₆)cycloalkyl-(C₃-C₆)cycloalkyl, (C₁-C₄)alkyl-(C₃-C₆)cycloalkyl, (C₁-C₄)alkoxy, (C₁-C₄)haloalkoxy, (C₁-C₄)alkoxyimino, (C₁-C₄)alkylthio, (C₁-C₄)haloalkylthio, (C₁-C₄)alkylsulphinyl, (C₁-C₄)haloalkylsulphinyl, (C₁-C₄)alkylsulphonyl, (C₁-C₄)haloalkylsulphonyl, (C₁-C₄)alkylsulphonyloxy, (C₁-C₄)alkylcarbonyl, (C₁-C₄)haloalkylcarbonyl, aminocarbonyl, (C₁-C₄)alkylaminocarbonyl, di- (C₁-C₄)alkylaminocarbonyl, (C₁-C₄)alkylsulphonylamino, (C₁-C₄)alkylamino, di-(C₁-C₄)alkylamino, aminosulphonyl, (C₁-C₄)alkylaminosulphonyl or di-(C₁-C₄)alkylaminosulphonyl,
or A is -O-CF₂-O- and, together with Q¹ and the carbon atom to which it is bonded, forms a five-membered ring where Q¹ is carbon,
W is halogen, and
Y is halogen, CO₂R¹ or NO₂, where R¹ is (C₁-C₆) -alkyl or (C₁-C₆) -haloalkyl,
**characterized in that**, in a first process step a), a compound Q-H in which Q is as defined above is reacted with an organozinc base of the structure (NR³R⁴)-Zn-R² or (NR³R⁴)₂-Zn in which R² is halogen or -O-pivaloyl and
R³ and R⁴ together form a -(CH₂)₄-, -(CH₂)₅- or-(CH₂)₂O(CH₂)₂- group, where each of these groups may optionally be substituted by 1, 2, 3 or 4 R⁵ radicals and R⁵ is selected from the group consisting of methyl, ethyl, n-propyl and i-propyl,
to give a compound of the formula (IIIa) or the formula (IIIb) 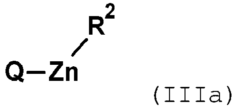
**Q-Zn-Q** (IIIb),
in which Q and R² each have the definitions given above,
and this compound of the formula (IIIa) or (IIIb) is reacted in a second process step b) with a compound of the formula (I) 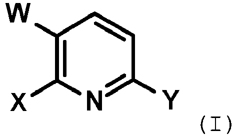 in which X is halogen and W and Y each have the definitions given above, in the presence of a catalyst, to give the compound of the formula (II).

2. Process according to Claim 1, **characterized in that**
Q is a structural element from the group of Q¹ to Q15 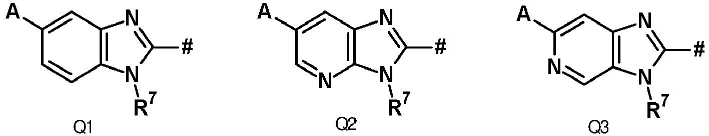 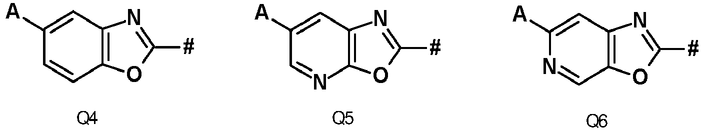 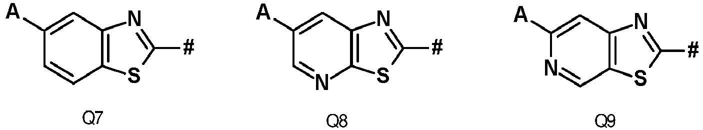 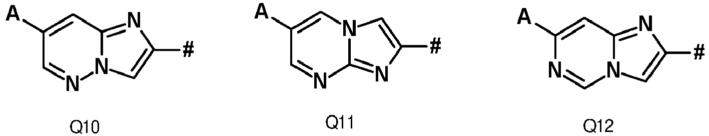 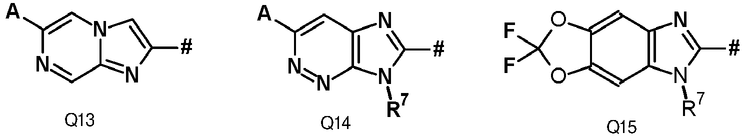
R⁷ is (C₁-C₄)alkyl, (C₁-C₄)haloalkyl, (C₁-C₄)alkoxy-(C₁-C₄)alkyl, (C₁-C₄)haloalkoxy-(C₁-C₄)alkyl, (C₁-C₄)alkylthio-(C₁-C₄)alkyl, (C₁-C₄)alkylsulphinyl-(C₁-C₄)alkyl, (C₁-C₄)alkylsulphonyl-(C₁-C₄)alkyl or (C₁-C₄)alkylcarbonyl-(C₁-C₄)alkyl,
A is fluorine, chlorine, bromine, fluoromethyl, difluoromethyl, trifluoromethyl, fluoroethyl (CH₂CFH₂, CHFCH₃), difluoroethyl (CF₂CH₃, CH₂CHF₂, CHFCFH₂), trifluoroethyl, (CH₂CF₃, CHFCHF₂, CF₂CFH₂), tetrafluoroethyl (CHFCF₃, CF₂CHF₂), pentafluoroethyl, trifluoromethoxy, difluorochloromethoxy, dichlorofluoromethoxy, trifluoromethylthio, trifluoromethylsulphinyl or trifluoromethylsulphonyl,
W is fluorine, chlorine or bromine,
R² is halogen, especially chlorine, bromine or iodine,
X is halogen, especially bromine or iodine, and
Y is fluorine, chlorine, bromine, CO₂R¹ or NO₂, where R¹ is (C₁-C₄) -alkyl.

3. Process according to either of Claims 1 and 2, **characterized in that**
Q is a structural element from the group of Q2, Q3, Q10, Q12, Q14 and Q15,
R⁷ is (C₁-C₄) alkyl or (C₁-C₄) alkoxy- (C₁-C₄) alkyl,
A is trifluoromethyl, fluoroethyl (CH₂CFH₂, CHFCH₃), difluoroethyl (CF₂CH₃, CH₂CHF₂, CHFCFH₂), trifluoroethyl, (CH₂CF₃, CHFCHF₂, CF₂CFH₂), tetrafluoroethyl (CHFCF₃, CF₂CHF₂), pentafluoroethyl, trifluoromethylthio, trifluoromethylsulphinyl or trifluoromethylsulphonyl,
W is fluorine or chlorine, especially fluorine,
R² is chlorine,
X is bromine or iodine, especially iodine, and
Y is chlorine, bromine, CO₂R¹ or NO₂, where R¹ is (C₁-C₄) -alkyl.

4. Process according to any of Claims 1 to 3, **characterized in that**
Q is the structural element Q3 or Q14,
R⁷ is methyl, ethyl, n-propyl or isopropyl, especially methyl,
A is trifluoromethyl,
W is fluorine,
R² is chlorine,
X is iodine, and
Y is chlorine, bromine, CO₂R¹ or NO₂, where R¹ is methyl.

5. Process according to any of Claims 1 to 4, **characterized in that** R³ and R⁴ together form a -(CH₂)₅- group substituted by 4 methyl groups.

6. Process according to any of Claims 1 to 5, **characterized in that** the organozinc base is a compound of the formula (V)
(V) (TMP)ₓ ZnCl₂₋ₓ
in which x is the number 1 or 2.

7. Process according to any of Claims 1 to 6, **characterized in that** the organozinc base is present in conjunction with an alkali metal halide or alkaline earth metal halide, preferably lithium chloride or magnesium chloride.

8. Process according to any of Claims 1 to 7, **characterized in that** the organozinc base is used in a total amount of 0.5 to 5 equivalents, based on the compound Q-H.

9. Process according to any of Claims 1 to 8, **characterized in that** the compound of the formula (I) is used in a total amount of 0.5 to 10.0 equivalents, based on the compound Q-H.

10. Process according to any of Claims 1 to 9, **characterized in that** the catalyst is a palladium compound.

11. Process according to any of Claims 1 to 10, **characterized in that** the catalyst is tetrakis(triphenylphosphine)palladium(0) .

12. Process according to any of Claims 1 to 11, **characterized in that** it is conducted in the presence of a solvent selected from the group consisting of tetrahydrofuran (THF), 1,4-dioxane, diethyl ether, diglyme, methyl tert-butyl ether (MTBE), tert-amyl methyl ether (TAME), 2-methyl-THF, toluene, xylenes, mesitylene, ethylene carbonate, propylene carbonate, N,N-dimethylacetamide, N,N-dimethylformamide (DMF), N-methylpyrrolidone (NMP), N-ethyl-2-pyrrolidone (NEP), N-butyl-2-pyrrolidone (NBP); N,N'-dimethylpropyleneurea (DMPU), halohydrocarbon, aromatic hydrocarbon, chlorohydrocarbon, tetrachloroethylene, tetrachloroethane, dichloropropane, methylene chloride, dichlorobutane, chloroform, carbon tetrachloride, trichloroethane, trichloroethylene, pentachloroethane, difluorobenzene, 1,2-dichloroethane, chlorobenzene, bromobenzene, dichlorobenzene, 1,2-dichlorobenzene, chlorotoluene, trichlorobenzene; 4-methoxybenzene, fluorinated aliphatic, fluorinated aromatic, trichlorotrifluoroethane, benzotrifluoride and 4-chlorobenzotrifluoride, or a mixture of at least two of these solvents with one another.

13. Process according to Claim 12, **characterized in that** the solvent is THF or N,N-dimethylformamide (DMF).

14. Process according to any of Claims 1 to 13, **characterized in that** process step a) is conducted at a temperature between 0°C and 80°C.

15. Process according to any of Claims 1 to 13, **characterized in that** process step b) is conducted at a temperature between 40°C and 90°C.

16. Compound of the structure 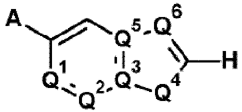 where
Q¹ is N or CR⁶,
Q² is N or CR⁶,
Q³ is N or C,
Q⁴ is O, S, N or NR⁷,
Q⁵ is N or C,
Q⁶ is N or CH,
R⁶ is hydrogen, (C₁-C₄)alkyl, (C₁-C₄)haloalkyl, (C₁-C₄)cyanoalkyl, (C₁-C₄)hydroxyalkyl, (C₁-C₄)alkoxy-(C₁-C₄)alkyl, (C₁-C₄)haloalkoxy-(C₁-C₄)alkyl, (C₂-C₄)alkenyl, (C₂-C₄)alkenyloxy-(C₁-C₄)alkyl, (C₂-C₄)haloalkenyloxy-(C₁-C₄)alkyl, (C₂-C₄)haloalkenyl, (C₂-C₄)cyanoalkenyl, (C₂-C₄)alkynyl, (C₂-C₄)alkynyloxy-(C₁-C₄)alkyl, (C₂-C₄)haloalkynyl, (C₃-C₆)cycloalkyl, (C₃-C₆)cycloalkyl-(C₃-C₆)cycloalkyl, (C₁-C₄)alkyl-(C₃-C₆)cycloalkyl, halo(C₃-C₆)cycloalkyl, (C₁-C₄)alkylthio-(C₁-C₄)alkyl, (C₁-C₄)alkylsulphinyl-(C₁-C₄)alkyl, (C₁-C₄)alkylsulphonyl-(C₁-C₄)alkyl or (C₁-C₄)alkylcarbonyl-(C₁-C₄)alkyl,
R⁷ is (C₁-C₄)alkyl, (C₁-C₄)haloalkyl, (C₁-C₄)cyanoalkyl, (C₁-C₄)hydroxyalkyl, (C₁-C₄)alkoxy-(C₁-C₄)alkyl, (C₁-C₄)haloalkoxy-(C₁-C₄)alkyl, (C₂-C₄)alkenyl, (C₂-C₄)alkenyloxy-(C₁-C₄)alkyl, (C₂-C₄)haloalkenyloxy-(C₁-C₄)alkyl, (C₂-C₄)haloalkenyl, (C₂-C₄)cyanoalkenyl, (C₂-C₄)alkynyl, (C₂-C₄)alkynyloxy-(C₁-C₄)alkyl, (C₂-C₄)haloalkynyl, (C₃-C₆)cycloalkyl, (C₃-C₆)cycloalkyl-(C₃-C₆)cycloalkyl, (C₁-C₄)alkyl- (C₃-C₆)cycloalkyl, halo(C₃-C₆)cycloalkyl, (C₁-C₄)alkylthio-(C₁-C₄)alkyl, (C₁-C₄)alkylsulphinyl-(C₁-C₄)alkyl, (C₁-C₄)alkylsulphonyl-(C₁-C₄)alkyl or (C₁-C₄)alkylcarbonyl-(C₁-C₄)alkyl, and
A is cyano, (C₁-C₄)alkyl, (C₁-C₄)haloalkyl, (C₂-C₄)alkenyl, (C₂-C₄)haloalkenyl, (C₂-C₄)alkynyl, (C₂-C₄)haloalkynyl, (C₃-C₆)cycloalkyl, (C₃-C₆)cycloalkyl-(C₃-C₆)cycloalkyl, (C₁-C₄)alkyl-(C₃-C₆)cycloalkyl, (C₁-C₄)alkoxy, (C₁-C₄)haloalkoxy, (C₁-C₄)alkoxyimino, (C₁-C₄)alkylthio, (C₁-C₄)haloalkylthio, (C₁-C₄)alkylsulphinyl, (C₁-C₄)haloalkylsulphinyl, (C₁-C₄)alkylsulphonyl, (C₁-C₄)haloalkylsulphonyl, (C₁-C₄)alkylsulphonyloxy, (C₁-C₄)alkylcarbonyl, (C₁-C₄)haloalkylcarbonyl, aminocarbonyl, (C₁-C₄)alkylaminocarbonyl, di-(C₁-C₄)alkylaminocarbonyl, (C₁-C₄)alkylsulphonylamino, (C₁-C₄)alkylamino, di-(C₁-C₄)alkylamino, aminosulphonyl, (C₁-C₄)alkylaminosulphonyl or di-(C₁-C₄)alkylaminosulphonyl,
or A is -O-CF₂-O- and, together with Q¹ and the carbon atom to which it is bonded, forms a five-membered ring where Q¹ is carbon.

17. Compound of the formula (IIIa) 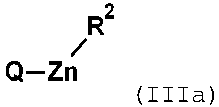 in which
Q is a structural element from the group of Q¹ to Q15,
R⁷ is (C₁-C₄)alkyl, (C₁-C₄)haloalkyl, (C₁-C₄)alkoxy-(C₁-C₄)alkyl, (C₁-C₄)haloalkoxy-(C₁-C₄)alkyl, (C₁-C₄)alkylthio-(C₁-C₄)alkyl, (C₁-C₄)alkylsulphinyl-(C₁-C₄)alkyl, (C₁-C₄)alkylsulphonyl-(C₁-C₄)alkyl or (C₁-C₄)alkylcarbonyl-(C₁-C₄)alkyl,
A is fluorine, chlorine, bromine, fluoromethyl, difluoromethyl, trifluoromethyl, fluoroethyl (CH₂CFH₂, CHFCH₃), difluoroethyl (CF₂CH₃, CH₂CHF₂, CHFCFH₂), trifluoroethyl, (CH₂CF₃, CHFCHF₂, CF₂CFH₂), tetrafluoroethyl (CHFCF₃, CF₂CHF₂), pentafluoroethyl, trifluoromethoxy, difluorochloromethoxy, dichlorofluoromethoxy, trifluoromethylthio, trifluoromethylsulphinyl or trifluoromethylsulphonyl, and
R² is halogen, especially chlorine, bromine or iodine.

18. Compound of the formula (IIIb)
**Q-Zn-Q** (IIIb)
in which
Q is a structural element from the group of Q1 to Q15,
R⁷ is (C₁-C₄)alkyl, (C₁-C₄)haloalkyl, (C₁-C₄)alkoxy-(C₁-C₄)alkyl, (C₁-C₄)haloalkoxy-(C₁-C₄)alkyl, (C₁-C₄)alkylthio-(C₁-C₄)alkyl, (C₁-C₄)alkylsulphinyl-(C₁-C₄)alkyl, (C₁-C₄)alkylsulphonyl-(C₁-C₄)alkyl or (C₁-C₄)alkylcarbonyl-(C₁-C₄)alkyl, and
A is fluorine, chlorine, bromine, fluoromethyl, difluoromethyl, trifluoromethyl, fluoroethyl (CH₂CFH₂, CHFCH₃), difluoroethyl (CF₂CH₃, CH₂CHF₂, CHFCFH₂), trifluoroethyl, (CH₂CF₃, CHFCHF₂, CF₂CFH₂) , tetrafluoroethyl (CHFCF₃, CF₂CHF₂), pentafluoroethyl, trifluoromethoxy, difluorochloromethoxy, dichlorofluoromethoxy, trifluoromethylthio, trifluoromethylsulphinyl or trifluoromethylsulphonyl.

19. Compound of the formula (II) 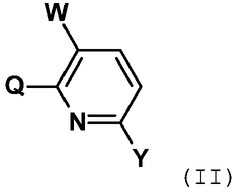 in which
Q is a structural element from the group of Q¹ to Q15,
R⁷ is (C₁-C₄)alkyl, (C₁-C₄)haloalkyl, (C₁-C₄)alkoxy-(C₁-C₄)alkyl, (C₁-C₄)haloalkoxy-(C₁-C₄)alkyl, (C₁-C₄)alkylthio-(C₁-C₄)alkyl, (C₁-C₄)alkylsulphinyl-(C₁-C₄)alkyl, (C₁-C₄)alkylsulphonyl-(C₁-C₄)alkyl or (C₁-C₄)alkylcarbonyl-(C₁-C₄)alkyl,
A is fluorine, chlorine, bromine, fluoromethyl, difluoromethyl, trifluoromethyl, fluoroethyl (CH₂CFH₂, CHFCH₃), difluoroethyl (CF₂CH₃, CH₂CHF₂, CHFCFH₂), trifluoroethyl, (CH₂CF₃, CHFCHF₂, CF₂CFH₂), tetrafluoroethyl (CHFCF₃, CF₂CHF₂), pentafluoroethyl, trifluoromethoxy, difluorochloromethoxy, dichlorofluoromethoxy, trifluoromethylthio, trifluoromethylsulphinyl or trifluoromethylsulphonyl,
W is fluorine or bromine, and
Y is fluorine, chlorine, bromine, CO₂R¹ or NO₂, where R¹ is (C₁-C₄)-alkyl.

## Revendications

1. Procédé pour la préparation de composés de formule (II) 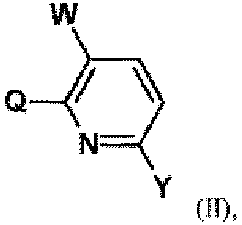 dans laquelle
Q représente un élément structural 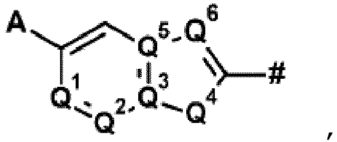
le signe # indiquant la liaison au reste de la molécule et
Q¹ représentant N ou CR⁶,
Q² représentant N ou CR⁶,
Q³ représentant N ou C,
Q⁴ représentant O, S, N ou NR⁷,
Q⁵ représentant N ou C,
Q⁶ représentant N ou CH,
R⁶ représentant hydrogène, (C₁-C₄)alkyle, (C₁-C₄)halogénoalkyle, (C₁-C₄)cyanoalkyle, (C₁-C₄)hydroxyalkyle, (C₁-C₄)alcoxy-(C₁-C₄)alkyle, (C₁-C₄)halogénoalcoxy-(C₁-C₄)alkyle, (C₂-C₄)alcényle, (C₂-C₄)alcényloxy-(C₁-C₄)alkyle, (C₂-C₄)halogénoalcényloxy-(C₁-C₄)alkyle, (C₂-C₄)halogénoalcényle, (C₂-C₄)cyanoalcényle, (C₂-C₄)alcynyle, (C₂-C₄)alcynyloxy-(C₁-C₄)alkyle, (C₂-C₄)halogénoalcynyle, (C₃-C₆)cycloalkyle, (C₃-C₆)cycloalkyl-(C₃-C₆)cycloalkyle, (C₁-C₄)alkyl-(C₃-C₆)cycloalkyle, halogène(C₃-C₆)cycloalkyle, (C₁-C₄)alkylthio-(C₁-C₄)alkyle, (C₁-C₄)alkylsulfinyl-(C₁-C₄)alkyle, (C₁-C₄)alkylsulfonyl-(C₁-C₄)alkyle ou (C₁-C₄)alkylcarbonyl-(C₁-C₄)alkyle,
R⁷ représentant (C₁-C₄)alkyle, (C₁-C₄)halogénoalkyle, (C₁-C₄)cyanoalkyle, (C₁-C₄)hydroxyalkyle, (C₁-C₄)alcoxy-(C₁-C₄)alkyle, (C₁-C₄)halogénoalcoxy-(C₁-C₄)alkyle, (C₂-C₄)alcényle, (C₂-C₄)alcényloxy-(C₁-C₄)alkyle, (C₂-C₄)halogénoalcényloxy-(C₁-C₄)alkyle, (C₂-C₄)halogénoalcényle, (C₂-C₄)cyanoalcényle, (C₂-C₄)alcynyle, (C₂-C₄)alcynyloxy-(C₁-C₄)alkyle, (C₂-C₄)halogénoalcynyle, (C₃-C₆)cycloalkyle, (C₃-C₆)cycloalkyl-(C₃-C₆)cycloalkyle, (C₁-C₄)alkyl-(C₃-C₆)cycloalkyle, halogène(C₃-C₆)cycloalkyle, (C₁-C₄)alkylthio-(C₁-C₄)alkyle, (C₁-C₄)alkylsulfinyl-(C₁-C₄)alkyle, (C₁-C₄)alkylsulfonyl-(C₁-C₄)alkyle ou (C₁-C₄)alkylcarbonyl-(C₁-C₄)alkyle, et
A représentant hydrogène, cyano, halogène, (C₁-C₄)alkyle, (C₁-C₄)halogénoalkyle, (C₂-C₄)alcényle, (C₂-C₄)halogénoalcényle, (C₂-C₄)alcynyle, (C₂-C₄)halogénoalcynyle, (C₃-C₆)cycloalkyle, (C₃-C₆)cycloalkyl-(C₃-C₆)cycloalkyle, (C₁-C₄)alkyl-(C₃-C₆)cycloalkyle, (C₁-C₄)alcoxy, (C₁-C₄)halogénoalcoxy, (C₁-C₄)alcoxyimino, (C₁-C₄)alkylthio, (C₁-C₄)halogénoalkylthio, (C₁-C₄)alkylsulfinyle, (C₁-C₄)halogénoalkylsulfinyle, (C₁-C₄)alkylsulfonyle, (C₁-C₄)halogénoalkylsulfonyle, (C₁-C₄)alkylsulfonyloxy, (C₁-C₄)alkylcarbonyle, (C₁-C₄)halogénoalkylcarbonyle, aminocarbonyle, (C₁-C₄)alkylaminocarbonyle, di-(C₁-C₄) alkyl-aminocarbonyle, (C₁-C₄)alkylsulfonylamino, (C₁-C₄)alkylamino, di-(C₁-C₄)alkylamino, aminosulfonyle, (C₁-C₄)alkylaminosulfonyle ou di-(C₁-C₄)alkylaminosulfonyle,
ou A représentant -O-CF₂-O- et, conjointement avec Q¹ et l'atome de carbone auquel il est lié, formant un cycle à cinq chaînons, Q¹ représentant carbone,
W représentant halogène, et
Y représentant halogène, CO₂R¹ ou NO₂, R¹ représentant (C₁-C₆)-alkyle ou (C₁-C₆)-halogénoalkyle,
**caractérisé en ce que** dans une première étape de procédé a) un composé Q-H, dans lequel Q possède la signification mentionnée ci-dessus,
est transformé avec une base organométallique de zinc de structure (NR³R⁴)-Zn-R² ou (NR³R⁴)₂-Zn, dans laquelle
R² représente halogène ou -O-pivaloyle et
R³ et R⁴ forment ensemble un groupe -(CH₂)₄-,-(CH₂)₅- ou -(CH₂)₂O(CH₂)₂-, chacun de ces groupes pouvant éventuellement être substitué par 1, 2, 3 ou 4 radicaux R⁵ et R⁵ étant choisi dans le groupe constitué par méthyle, éthyle, n-propyle et i-propyle,
pour donner un composé de formule (IIIa) ou de formule (IIIb), 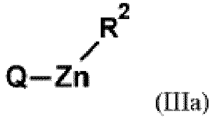
**Q-Zn-Q** (IIIb),
dans laquelle Q et R² à chaque fois possèdent les significations mentionnées ci-dessus,
et ce composé de formule (IIIa) ou (IIIb) dans une deuxième étape de procédé b) est transformé avec un composé de formule (I) 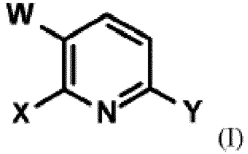 dans laquelle X représente halogène et W et Y possèdent à chaque fois les significations mentionnées ci-dessus, en présence d'un catalyseur pour donner le composé de formule (II).

2. Procédé selon la revendication 1, **caractérisé en ce que**
Q représente un élément structural de la série Q¹ à Q15, 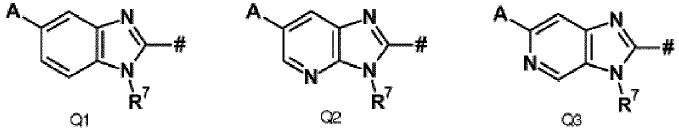 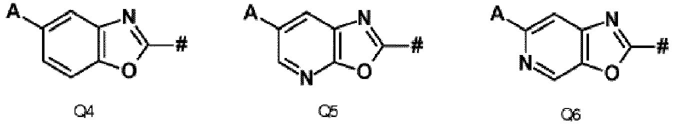 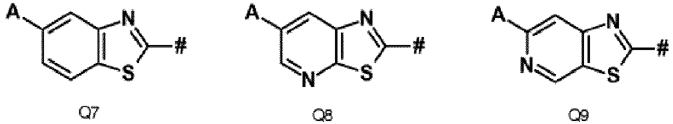 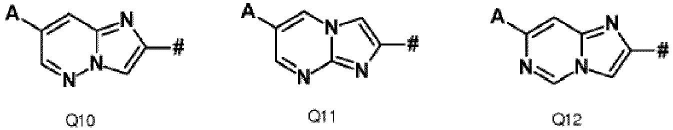 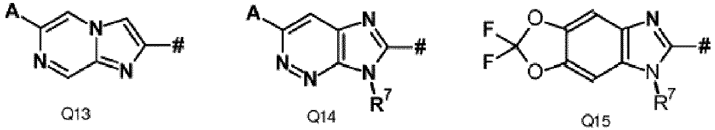
R⁷ représente (C₁-C₄)alkyle, (C₁-C₄)halogénoalkyle, (C₁-C₄)alcoxy-(C₁-C₄)alkyle, (C₁-C₄)halogénoalcoxy-(C₁-C₄)alkyle, (C₁-C₄)alkylthio-(C₁-C₄)alkyle, (C₁-C₄)alkylsulfinyl-(C₁-C₄)alkyle, (C₁-C₄)alkylsulfonyl-(C₁-C₄)alkyle ou (C₁-C₄)alkylcarbonyl-(C₁-C₄)alkyle,
A représente fluor, chlore, brome, fluorométhyle, difluorométhyle, trifluorométhyle, fluoroéthyle, (CH₂CFH₂, CHFCH₃), difluoréthyle (CF₂CH₃, CH₂CHF₂, CHFCFH₂), trifluoréthyle, (CH₂CF₃, CHFCHF₂, CF₂CFH₂), tétrafluoréthyle (CHFCF₃, CF₂CHF₂), pentafluoréthyle, trifluorométhoxy, difluorochlorométhoxy, dichlorofluorométhoxy, trifluorométhylthio, trifluorométhylsulfinyle ou trifluorométhylsulfonyle,
W représente fluor, chlore ou brome,
R² représente halogène, en particulier chlore, brome ou iode,
X représente halogène, en particulier brome ou iode, et
Y représente fluor, chlore, brome, CO₂R¹ ou NO₂, R¹ représentant (C₁-C₄)-alkyle.

3. Procédé selon l'une quelconque des revendications 1 et 2, **caractérisé en ce que**
Q représente un élément structural de la série Q2, Q3, Q10, Q12, Q14 ou Q15,
R⁷ représente (C₁-C₄) alkyle ou (C₁-C₄) alkyloxy- (C₁-C₄) alkyle,
A représente trifluorométhyle, fluoroéthyle (CH₂CFH₂, CHFCH₃), difluoroéthyle (CF₂CH₃, CH₂CHF₂, CHFCFH₂), trifluoréthyle, (CH₂CF₃, CHFCHF₂, CF₂CFH₂), tétrafluoréthyle (CHFCF₃, CF₂CHF₂), pentafluoroéthyle, trifluorométhylthio, trifluorométhylsulfinyle ou trifluorométhylsulfonyle,
W représente fluor ou chlore, en particulier fluor,
R² représente chlore,
X représente brome ou iode, en particulier iode, et
Y représente chlore, brome, CO₂R¹ ou NO₂, R¹ représentant (C₁-C₄)-alkyle.

4. Procédé selon l'une quelconque des revendications 1 à 3, **caractérisé en ce que**
Q représente l'élément structural Q3 ou Q14,
R⁷ représente méthyle, éthyle, n-propyle, iso-propyle, en particulier méthyle,
A représente trifluorométhyle,
W représente fluor,
R² représente chlore,
X représente iode, et
Y représente chlore, brome, CO₂R¹ ou NO₂, R¹ représentant méthyle.

5. Procédé selon l'une quelconque des revendications 1 à 4, **caractérisé en ce que**
R³ et R⁴ forment ensemble un groupe -(CH₂)₅-, qui est substitué par 4 groupes méthyle.

6. Procédé selon l'une quelconque des revendications 1 à 5, **caractérisé en ce que** la base organométallique de zinc est un composé de formule (V)
(V) (TMP)ₓ ZnCl₂₋ₓ ,
dans laquelle x représente le nombre 1 ou 2.

7. Procédé selon l'une quelconque des revendications 1 à 6, **caractérisé en ce que** la base organométallique de zinc est présente en combinaison avec un halogénure alcalin ou un halogénure alcalino-terreux, de préférence du chlorure de lithium ou du chlorure de magnésium.

8. Procédé selon l'une quelconque des revendications 1 à 7, **caractérisé en ce que** la base organométallique de zinc est utilisée en une quantité totale de 0,5 à 5 équivalents, par rapport au composé Q-H.

9. Procédé selon l'une quelconque des revendications 1 à 8, **caractérisé en ce que** le composé de formule (I) est utilisé en une quantité totale de 0,5 à 10,0 équivalents, par rapport au composé Q-H.

10. Procédé selon l'une quelconque des revendications 1 à 9, **caractérisé en ce que** le catalyseur est un composé du palladium.

11. Procédé selon l'une quelconque des revendications 1 à 10, **caractérisé en ce que** le catalyseur est le tétrakis(triphénylphosphine)palladium(0).

12. Procédé selon l'une quelconque des revendications 1 à 11, **caractérisé en ce qu'**il est mis en œuvre en présence d'un solvant qui est choisi dans le groupe constitué par le tétrahydrofuranne (THF), le 1,4-dioxanne, le diéthyléther, le diglyme, le méthyltertbutyléther (MTBE), le tert-amyl-méthyléther (TAME), le 2-méthyl-THF, le toluène, les xylènes, le mésitylène, le carbonate d'éthylène, le carbonate de propylène, le N,N-diméthylacétamide, le N,N-diméthylformamide (DMF), la N-méthylpyrrolidone (NMP), la N-éthyl-2-pyrrolidone (NEP), la N-butyl-2-pyrrolidone (NBP) ; la N,N'-diméthylpropylèneurée (DMPU), un halogénohydrocarbure, un hydrocarbure aromatique, un chlorohydrocarbure, le tétrachloroéthylène, le tétrachloroéthane, le dichloropropane, le chlorure de méthylène, le dichlorobutane, le chloroforme, le tétrachlorure de carbone, le trichloroéthane, le trichloréthylène, le pentachloroéthane, le difluorobenzène, le 1,2-dichloroéthane, le chlorobenzène, le bromobenzène, un dichlorobenzène, le 1,2-dichlorobenzène, un chlorotoluène, un trichlorobenzène ; le 4-méthoxybenzène, un composé aliphatique fluoré, un composé aromatique fluoré, le trichlorotrifluoroéthane, le trifluorométhylbenzène et le 4-chlorotrifluorométhylbenzène ou un mélange d'au moins deux de ces solvants entre eux.

13. Procédé selon la revendication 12, **caractérisé en ce que** le solvant est le THF ou le N,N-diméthylformamide (DMF).

14. Procédé selon l'une quelconque des revendications 1 à 13, **caractérisé en ce que** l'étape de procédé a) est mise en œuvre à une température comprise entre 0 °C et 80 °C.

15. Procédé selon l'une quelconque des revendications 1 à 13, **caractérisé en ce que** l'étape de procédé b) est mise en œuvre à une température comprise entre 40 °C et 90 °C.

16. Composé de structure 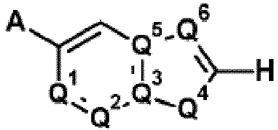
Q¹ représentant N ou CR⁶,
Q² représentant N ou CR⁶,
Q³ représentant N ou C,
Q⁴ représentant O, S, N ou NR⁷,
Q⁵ représentant N ou C,
Q⁶ représentant N ou CH,
R⁶ représentant hydrogène, (C₁-C₄)alkyle, (C₁-C₄)halogénoalkyle, (C₁-C₄)cyanoalkyle, (C₁-C₄)hydroxyalkyle, (C₁-C₄)alcoxy-(C₁-C₄)alkyle, (C₁-C₄)halogénoalcoxy-(C₁-C₄)alkyle, (C₂-C₄)alcényle, (C₂-C₄)alcényloxy-(C₁-C₄)alkyle, (C₂-C₄)halogénoalcényloxy-(C₁-C₄)alkyle, (C₂-C₄)halogénoalcényle, (C₂-C₄)cyanoalcényle, (C₂-C₄)alcynyle, (C₂-C₄)alcynyloxy-(C₁-C₄)alkyle, (C₂-C₄)halogénoalcynyle, (C₃-C₆)cycloalkyle, (C₃-C₆)cycloalkyl-(C₃-C₆)cycloalkyle, (C₁-C₄)alkyl-(C₃-C₆)cycloalkyle, halogéno(C₃-C₆)cycloalkyle, (C₁-C₄)alkylthio-(C₁-C₄)alkyle, (C₁-C₄)alkylsulfinyl-(C₁-C₄) alkyle, (C₁-C₄)alkylsulfonyl-(C₁-C₄)alkyle ou (C₁-C₄)alkylcarbonyl-(C₁-C₄)alkyle,
R⁷ représentant (C₁-C₄)alkyle, (C₁-C₄)halogénoalkyle, (C₁-C₄)cyanoalkyle, (C₁-C₄)hydroxyalkyle, (C₁-C₄)alcoxy-(C₁-C₄)alkyle, (C₁-C₄)halogénoalcoxy-(C₁-C₄)alkyle, (C₂-C₄)alcényle, (C₂-C₄)alcényloxy-(C₁-C₄)alkyle, (C₂-C₄)halogénoalcényloxy-(C₁-C₄)alkyle, (C₂-C₄)halogénoalcényle, (C₂-C₄)cyanoalcényle, (C₂-C₄)alcynyle, (C₂-C₄)alcynyloxy-(C₁-C₄)alkyle, (C₂-C₄)halogénoalcynyle, (C₃-C₆)cycloalkyle, (C₃-C₆)cycloalkyl-(C₃-C₆)cycloalkyle, (C₁-C₄)alkyl-(C₃-C₆)cycloalkyle, halogène(C₃-C₆)cycloalkyle, (C₁-C₄)alkylthio-(C₁-C₄)alkyle, (C₁-C₄)alkylsulfinyl-(C₁-C₄)alkyle, (C₁-C₄)alkylsulfonyl-(C₁-C₄)alkyle ou (C₁-C₄)alkylcarbonyl-(C₁-C₄)alkyle, et
A représentant cyano, (C₁-C₄)alkyle, (C₁-C₄)halogénoalkyle, (C₂-C₄)alcényle, (C₂-C₄)halogénoalcényle, (C₂-C₄)alcynyle, (C₂-C₄)halogénoalcynyle, (C₃-C₆)cycloalkyle, (C₃-C₆)cycloalkyl-(C₃-C₆)cycloalkyle, (C₁-C₄)alkyl-(C₃-C₆) cycloalkyle, (C₁-C₄)alcoxy, (C₁-C₄)halogénoalcoxy, (C₁-C₄)alcoxyimino, (C₁-C₄)alkylthio, (C₁-C₄)halogénoalkylthio, (C₁-C₄)alkylsulfinyle, (C₁-C₄)halogénoalkylsulfinyle, (C₁-C₄)alkylsulfonyle, (C₁-C₄)halogénoalkylsulfonyle, (C₁-C₄)alkylsulfonyloxy, (C₁-C₄)alkylcarbonyle, (C₁-C₄)halogénoalkylcarbonyle, aminocarbonyle, (C₁-C₄)alkylaminocarbonyle, di-(C₁-C₄)alkyl-aminocarbonyle, (C₁-C₄)alkylsulfonylamino, (C₁-C₄)alkylamino, di-(C₁-C₄)alkylamino, aminosulfonyle, (C₁-C₄)alkylaminosulfonyle ou di-(C₁-C₄)alkylaminosulfonyle,
ou A représentant -O-CF₂-O- et, conjointement avec Q¹ et l'atome de carbone auquel il est lié, formant un cycle à cinq chaînons, Q¹ représentant carbone.

17. Composé de formule (IIIa) 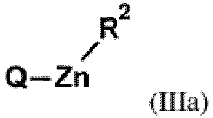 dans laquelle
Q représente un élément structural de la série Q1 à Q15,
R⁷ représente (C₁-C₄)alkyle, (C₁-C₄)halogénoalkyle, (C₁-C₄)alcoxy-(C₁-C₄)alkyle, (C₁-C₄)halogénoalcoxy-(C₁-C₄)alkyle, (C₁-C₄)alkylthio-(C₁-C₄)alkyle, (C₁-C₄)alkylsulfinyl-(C₁-C₄)alkyle, (C₁-C₄)alkylsulfonyl-(C₁-C₄)alkyle ou (C₁-C₄)alkylcarbonyl-(C₁-C₄)alkyle,
A représente fluor, chlore, brome, fluorométhyle, difluorométhyle, trifluorométhyle, fluoroéthyle, (CH₂CFH₂, CHFCH₃), difluoréthyle (CF₂CH₃, CH₂CHF₂, CHFCFH₂), trifluoréthyle, (CH₂CF₃, CHFCHF₂, CF₂CFH₂), tétrafluoréthyle (CHFCF₃, CF₂CHF₂), pentafluoréthyle, trifluorométhoxy, difluorochlorométhoxy, dichlorofluorométhoxy, trifluorométhylthio, trifluorométhylsulfinyle ou trifluorométhylsulfonyle, et
R² représente halogène, en particulier chlore, brome ou iode.

18. Composé de formule (IIIb)
Q-Zn-Q (IIIb)
dans laquelle
Q représente un élément structural de la série Q1 à Q15,
R⁷ représente (C₁-C₄)alkyle, (C₁-C₄)halogénoalkyle, (C₁-C₄)alcoxy-(C₁-C₄) alkyle, (C₁-C₄)halogénoalcoxy-(C₁-C₄)alkyle, (C₁-C₄)alkylthio-(C₁-C₄)alkyle, (C₁-C₄)alkylsulfinyl-(C₁-C₄)alkyle, (C₁-C₄)alkylsulfonyl-(C₁-C₄)alkyle ou (C₁-C₄)alkylcarbonyl-(C₁-C₄)alkyle, et
A représente fluor, chlore, brome, fluorométhyle, difluorométhyle, trifluorométhyle, fluoroéthyle, (CH₂CFH₂, CHFCH₃) , difluoréthyle (CF₂CH₃, CH₂CHF₂, CHFCFH₂), trifluoréthyle, (CH₂CF₃, CHFCHF₂, CF₂CFH₂), tétrafluoréthyle (CHFCF₃, CF₂CHF₂), pentafluoréthyle, trifluorométhoxy, difluorochlorométhoxy, dichlorofluorométhoxy, trifluorométhylthio, trifluorométhylsulfinyle ou trifluorométhylsulfonyle.

19. Composé de formule (II) 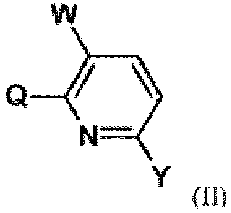 dans laquelle
Q représente un élément structural de la série Q1 à Q15,
R⁷ représente (C₁-C₄)alkyle, (C₁-C₄)halogénoalkyle, (C₁-C₄)alcoxy-(C₁-C₄)alkyle, (C₁-C₄)halogénoalcoxy-(C₁-C₄)alkyle, (C₁-C₄)alkylthio-(C₁-C₄)alkyle, (C₁-C₄)alkylsulfinyl-(C₁-C₄)alkyle, (C₁-C₄)alkylsulfonyl-(C₁-C₄)alkyle ou (C₁-C₄)alkylcarbonyl-(C₁-C₄)alkyle,
A représente fluor, chlore, brome, fluorométhyle, difluorométhyle, trifluorométhyle, fluoroéthyle, (CH₂CFH₂, CHFCH₃), difluoréthyle (CF₂CH₃, CH₂CHF₂, CHFCFH₂), trifluoréthyle, (CH₂CF₃, CHFCHF₂, CF₂CFH₂), tétrafluoréthyle (CHFCF₃, CF₂CHF₂), pentafluoréthyle, trifluorométhoxy, difluorochlorométhoxy, dichlorofluorométhoxy, trifluorométhylthio, trifluorométhylsulfinyle ou trifluorométhylsulfonyle,
W représente fluor ou brome, et
Y représente fluor, chlore, brome, CO₂R¹ ou NO₂, R¹ représentant (C₁-C₄)-alkyle.
